(19) **Europäisches Patentamt / European Patent Office / Office européen des brevets**

(11) **EP 4 041 069 B1**

(12) **EUROPEAN PATENT SPECIFICATION**

(45) Date of publication and mention
of the grant of the patent:
**08.10.2025   Bulletin 2025/41**

(21) Application number: **21701408.3**

(22) Date of filing: **12.01.2021**

(51) International Patent Classification (IPC):
**A61B 5/00** *(2006.01)*        **A61B 5/22** *(2006.01)*
**A63B 23/20** *(2006.01)*

(52) Cooperative Patent Classification (CPC):
**A61B 5/4337; A61B 5/22; A63B 23/20**

(86) International application number:
**PCT/EP2021/050493**

(87) International publication number:
**WO 2021/144270 (22.07.2021 Gazette 2021/29)**

(54) **SYSTEM FOR MONITORING PELVIC FLOOR MUSCLE CONTRACTIONS**

SYSTEM ZUR ÜBERWACHUNG DER KONTRAKTIONEN DER BECKENBODENMUSKULATUR

SYSTÈME DE SUIVI DES CONTRACTIONS DES MUSCLES DU PLANCHER PELVIEN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.01.2020   GB 202000633**

(43) Date of publication of application:
**17.08.2022   Bulletin 2022/33**

(73) Proprietor: **X6 Innovations**
**75008 Paris (FR)**

(72) Inventors:
- **RODIONOV, Artem**
**75008 PARIS (FR)**
- **HAOUDI, Cyril**
**75008 PARIS (FR)**

(74) Representative: **Fidal Innovation**
**4-6 avenue d'Alsace**
**92400 Courbevoie (FR)**

(56) References cited:
**WO-A1-2012/079127        WO-A1-2017/118461
CN-A- 110 538 432        CN-U- 208 145 360
JP-A- 2002 143 133**

- **ANONYMOUS: "Perifit", INTERNET CITATION, 10
December 2019 (2019-12-10), pages 1 - 13,
XP009525989, Retrieved from the Internet
<URL:https://web.archive.org/web/
20200411050131/https://perifit.co/> [retrieved on
20200714]**

## Description

### FIELD OF THE INVENTION

The present invention generally relates to a pelvic floor kegel exercising system. More particularly, the present invention relates to a pelvic floor muscles (PFM) contraction monitoring system.

### BACKGROUND OF THE INVENTION

Pelvic floor muscles stretch from the pubic bone to the tailbone, surrounding the opening of the urethra, vagina and bowel. The pelvic floor muscles (PFM) are a crucial structure as they support the organs of the pelvic region, i.e., the bladder, rectum, and uterus. The pelvic muscles become damaged or weakened through childbirth, lack of use, age, or other reasons. Many pathological conditions, such as cystocele (hernial protrusion of the urinary bladder through the vaginal wall), urinary incontinence, rectocele (hernial protrusion of part of the rectum into the vagina), uterine prolapse (protrusion of the uterus through the vaginal orifice), and bladder and sexual dysfunctions, may be caused by a weakened condition of the pelvic floor muscles.

Therefore, various pelvic floor muscle exercises and devices have been developed for strengthening PFMs, particularly muscles surrounding the urethra. These exercises generally involve simple clench-and-release exercises that could make the muscles of the pelvic floor stronger. However, merely squeezing the pelvic muscles without any feedback information on the contraction is not effective. Indeed, only a few women could do kegel exercises correctly. Also, most women cannot identify the pelvic region's muscles to exercise and cannot monitor their progress and/or results. Finally, numerous studies have been showing a correlation between the ability to receive visual feedback of muscle contractions (biofeedback) and the efficiency of pelvic floor rehabilitation therapy.

Several methods of monitoring and detecting incorrect PFM contractions have been developed. One method involves manual detection by a trained health professional. Another method involves mechanical detection by observing a movement of geisha balls, vaginal cones, or similar objects inserted in the vaginal canal. During an incorrect contraction, the device is pushed out of the vagina. The patient must thus visually spot the outside movement of the device to detect an incorrect PFM contraction. Other devices such as electronic imagery devices, EMG biofeedback probes with multiple sensors, and probes using embedded accelerometers have also been developed for detecting incorrect pelvic floor muscles contractions.

Document US2015196802 describes a medical device with pressure sensors for detecting muscle contraction, which is placed inside of the body, for tracking and guiding pelvic muscle exercise. A microcontroller is used for processing data from the sensors and which are displayed to the user interface. The method and device may be used by men and women for treating urinary incontinence, sexual dysfunction, and other pelvic conditions.

US publication application No. US2018/0264317A1 describes a pelvic floor muscle exercise system and detection device which is placed inside of the body and has two pressure sensors used for detection of muscle contraction. A microcontroller is used for processing data from the sensors and then displays any incorrect muscle contractions to the user's smartphone. The physical training system for pelvic floor muscle comprises a processor capable of providing at least one optional exercising setting to the user, which includes an exercising time setting, an exercising intensity setting, an exercising frequency setting, an exercising guidance setting, an exercising auxiliary setting and an exercising feedback setting. The document CN110538432 A discloses a training method and system of portable pelvic floor muscle training device.

The present invention, however, is directed to a device for monitoring pelvic floor muscle contraction, which provides the user with reliable information on the quality and types of contractions and thus can efficiently assist the user in an exercise routine of pelvic floor muscles.

### SUMMARY OF THE INVENTION

The present invention thus relates to a reliable a system for monitoring PFM contractions in a subject and more particularly for detecting occurrences of incorrect pelvic floor muscle (PFM) contractions or Valsalva contractions in said subject. The above clause, *"incorrect pelvic floor muscle contractions or Valsalva contractions",* is to be understood as encompassing any maneuver, voluntary or not, that involves an increase of pressure in the subject abdomen, i.e. an intra-abdominal pressure increase.

According to the invention, it is provided a system for monitoring pelvic floor muscle (PFM) contractions performed by a subject, notably a female individual as defined in claim 1

Thereby, such a system can detect, besides a PFM contraction, any contraction that involves an intra-abdominal pressure increase, and in particular Valsalva contraction, and such system provides a relevant notification to the user. Indeed, when exercising properly pelvic floor muscles, it is desired to contract the pelvic floor muscle while maintaining the contractions of other muscles in the abdomen at their minimum. Advantageously, using two distinct sensors in respective two bulbous shape sections helps to distinguish an intra-abdominal contraction reliably from a PFM contraction. The two sensors are decoupled. Studies show that contractions involving Intra-Abdominal Pressure greater than Vaginal Pressure have a negative impact on prolapse development in patients and decrease in patient's pelvic floor functions. Therefore, by monitoring the difference between vaginal and intra-abdominal pressures and keeping it positive we achieve a safer and more efficient treatment of pelvic floor muscles.

It has been found that some female individuals do not practice PFM properly after childbirth recovery or for any health purpose. Indeed, a healthy PFM contraction should not involve an excessive concurrent intra-abdominal pressure. Concurrent intra-abdominal contraction may happen in case of improperly exercising PFM contraction without the subject being aware. Therefore, the proposed probe and system help the subject to practice healthy contractions and avoid inefficient concurrent intra-abdominal contractions.

The promoted system provides reliable information on the muscle groups involved in the contraction and the overall quality of the contractions.

According to an embodiment of the invention, the pelvic floor probe device extends along a main axis (X), wherein the first sensor exhibits a main direction of sensing along a first transverse direction (W1) and the second sensor exhibits a main direction of sensing along a second transverse direction (Y2), wherein said second transverse direction is substantially perpendicular to the first transverse direction, and preferably, first and second transverse direction (W1,Y2) are substantially perpendicular to the main axis (X). Such an 'orthogonal' arrangement is relevant for improving the sensing of PFM, and for improving the sensing of intra-abdominal contraction. Decoupling between the two sensors is also increased

According to an embodiment of the invention, said first section (106) is separated from said second section (108) by a waist section (107) said waist section having a cross-section (D7) in size less than 75% 75% of the size of the cross-section (D6) of said first section (106) and wherein the waist section exhibits flexure compliance to allow a misalignment of said second section (108) relative to the said first section (106). This improves the comfort for the user during insertion and exercising. Furthermore, decoupling of sensing areas is further improved by the waist section.

According to an embodiment of the invention, the pelvic floor probe device (100) comprises a third section (104) arranged at the proximal end of the device, and the third section (104) is separated from the first section (106) by a base waist portion (105). We thereby provide good retention of the device during exercising, particularly relevant in case of improper/suboptimal contraction with intra-abdominal pressure increase.

According to an embodiment of the invention, the third section (104) has an oblong transverse cross-section with a larger dimension along a base orientation noted WO, which is parallel to first transverse direction W1, whereby the user is induced to place the device with base orientation WO aligned with her antero-posterior direction. This arrangement provides general indexation around the main axis, securing thereby the preferred sensing directions.

According to an embodiment of the invention, the third section has an oblong transverse cross-section with a larger dimension along a base orientation noted WO, which is parallel to second transverse direction Y2, whereby the user is induced to place the device with base orientation Y0 aligned with her anteroposterior direction. This arrangement provides another general indexation around the main axis.

According to an embodiment of the invention, the pelvic floor probe device (100) comprises a first battery cell lodged in the first section (106) and a second battery cell lodged in the second section (108). Thereby optimum use of available space in the device, excellent autonomy without bothering about recharging. According to one particular option, each battery cell is a lithium coin battery. According to one particular option, CR2032 coin batteries are chosen as a best compromise regarding capacity versus size. According to one particular option, the battery cells are non-rechargeable battery cells.

According to an embodiment of the invention, first section (106) and second section (108) are attached together by a link ring (6) arranged at the waist section (107), the link ring (6) being encompassed by a collar (7). This arrangement provides an improved fit with subject anatomy and ease of assembly and control of the flexure joint.

According to an embodiment of the invention, the first section (106) comprises two complementary first shells (11,12), which, when assembled, form the bulbous body, with a first assembly joint arranged generally on a plane perpendicular to the first transverse direction **W1**. This provides ease of assembly of the items comprised within the first section, i.e. sensor and battery. There may be provided functional play between the two first shells.

According to an embodiment of the invention, the second section (108) comprises two complementary second shells (21,22), which, when assembled, form the bulbous body, with a second assembly joint arranged generally on a plane perpendicular to the second transverse direction **Y2**. As per first section, this favors ease of assembly and integration for the second section and items located therein.

According to an embodiment of the invention, the first section (106) exhibits a substantially circular cross section (D6), with an outer diameter no greater than 30 mm, preferably no greater than 28 mm. Advantageously, such small size is user friendly and convenient.

According to an embodiment of the invention, the said first and second sensors are electronic sensors chosen among electromechanical sensors or pressure sensors or force sensors. Reliable and cost-effective solution.

According to an embodiment of the invention, the first section is centered on a first axial position located at a first distance from the distal end of said probe body, the first distance D1 being comprised between 6 and 8 cm, preferably 7cm, and the second section is centered on a second axial position located at a second distance from the distal end of said probe body, the second distance D2 being comprised between 2,5 and 3,5 cm, preferably 3cm. Optimized positions for sensing PFM contraction and independently intra-abdominal contraction.

According to an embodiment of the invention, the device may comprise a fluid-tight envelope (8), said envelope being made of a biocompatible elastomer. The fluid-tight envelope can be preferably medical-grade silicon.

Further embodiments are laid down in claims 14-19

According to an embodiment of the invention, the data processing module is further configured to process the registered set of differential pressures data into a second characterizing function CF2 defined as follows:

$$CF2 = \frac{1}{1 + EXP\lceil c2x(\Delta Pmz(k) / \Delta Pdt(k) - c1x)\rceil}$$ ('EXP' is the exponential function).

And a cost optimization algorithm is defined according to the following cost function:

$$Cost(c_{1x}, c_{2x}) = ||CF2 - \delta i||$$

wherein the function ||*|| is a metric distance function and

- said data processing module being further configured to store the optimal parameters c1 and c2 as obtained from the optimization algorithm,
- an interface in communication with the data processing module, said interface being configured to receive data from the data processing module and notify an occurrence of an incorrect contraction when $$CF2(j) = \frac{1}{1 + EXP\lceil c2(\Delta Pmz(i) / \Delta Pdt(i) - c1)\rceil}$$ is above 0,5. ('EXP' is the exponential function).

The parameters c1 and c2 are therefore further personalized with regard to a particular user.

According to an example not falling within the scope of the claims, there is proposed a pelvic floor probe device (100) configured to be positioned within the vaginal canal and in contact with the internal vaginal surface of the subject, said probe comprising a body with a distal end and proximal end, an external surface, and at least two sections, a first section (106) being at the proximal end of the probe for contacting the midzone area of the vaginal canal and a second section (108) being positioned at the distal end of the probe for contacting the distal zone of the vaginal canal, each of said first and second sections having a bulbous shape, the first section comprising at least a first sensor adapted for measuring the pressure applied by the internal vaginal surface to the external surface of the probe at the midzone area of the vaginal canal, and the second section comprising at least a second sensor adapted for measuring the pressure applied by the internal vaginal surface to the external surface of the probe at a distal zone, wherein the pelvic floor probe device (100) extends along a main axis (X), wherein the first sensor exhibits a main direction of sensing along a first transverse direction (W1) and the second sensor exhibits a main direction of sensing along a second transverse direction (Y2), wherein said second transverse direction is substantially perpendicular to the first transverse direction, and preferably, first and second transverse directions (W1,Y2) are substantially perpendicular to the main axis (X).

According to this arrangement, said probe device is advantageously used to determine a healthy PFM contraction from any contraction that involves an excessive intra-abdominal pressure increase. Two different main directions of sensing have been found relevant to optimize sensing, independently for PFM contraction alone and intra-abdominal pressure increase.

**BRIEF DESCRIPTION OF DRAWINGS**

**FIG. 1** exemplarily illustrates a perspective view of a pelvic floor probe device, according to an embodiment of the present invention.

**FIG. 2** exemplarily illustrates an environment where the pelvic floor probe device is having a data processing module in communication with a user device, according to an embodiment of the present invention.

**FIG. 3** exemplarily illustrates a flowchart of a method of evaluating pelvic floor muscle (PFM) exercise performed by a user, according to an example not falling within the scope of the claims.

**FIG. 4** is a perspective view of the device, with partial cutaways.

**FIG. 5** exemplarily illustrates time charts of relative pressure values.

**FIG. 6** exemplarily illustrates more in detail a link ring attaching first and second sections.

**FIG. 7** is another illustrative perspective view encompassing the link ring and the second section.

**FIG. 8** shows a schematic elevation view of the device.

**FIG. 9** shows an exemplary block diagram.

**FIG. 10** shows a front view of the device.

## DETAILED DESCRIPTION

### Overview and probe device construction

The present invention is directed to a pelvic floor muscle (PFM) contraction monitoring system, device of evaluating pelvic floor muscle (PFM) exercise performed by a subject, and particularly for detecting incorrect and/or suboptimal PFM contraction or Valsalva contraction.

The pelvic floor contractions monitoring system comprises a probe, a data processing module, and a user interface in communication with the data processing module. The probe is adapted to position within a pelvic cavity of the user.

The probe comprises one or more sections including, a first section and a second section. The probe is incorporated with one or more groups of sensors, which include a first group of sensors and a second group of sensors. The first section and the second section are seated in contact with tissues of the pelvic cavity. The first section contains the first group of sensors, positioned within a midzone area of the pelvic cavity. The second section contains the second group of sensors, positioned within a distal zone of the pelvic cavity.

The first group of sensors and the second group of sensors are placed at a relative distance of approximately 3 cm to 7 cm deep inside the subject's vagina. The first group of sensors and second group of sensors contain at least one pressure sensor or force sensor. Therefore, the first group of sensors and the second group of sensors are configured to detect the pressure applied on the probe surface by the internal surface of the vagina.

The user interface in communication with the data processing module 202 is configured to provide a set of targets to perform PFM exercise. The targets include, but are not limited to, contraction start and end flags.

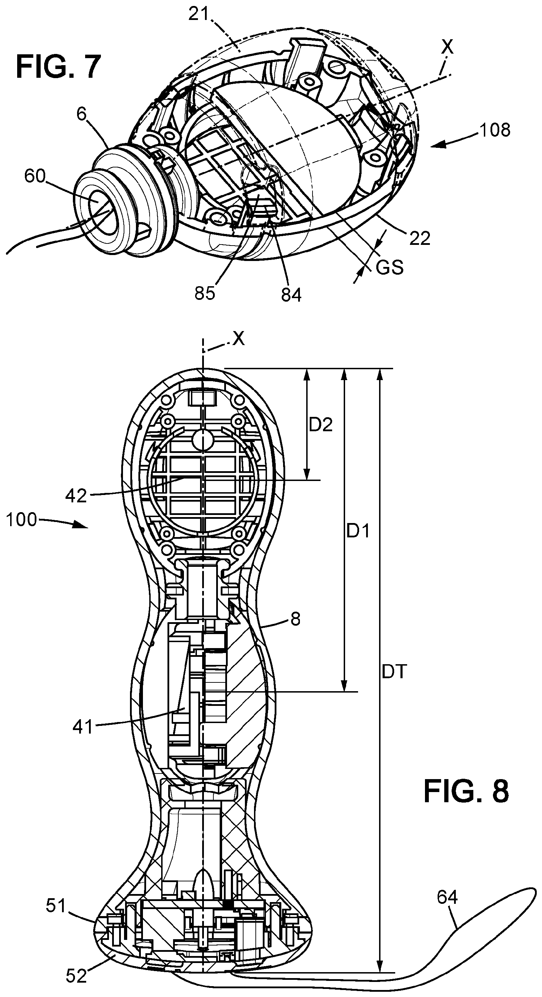

As shown in the figures, the pelvic floor probe device **100** extends along a main axis **X.** As apparent from Figure 8, the length of the body **DT** is comprised between 10 cm and 12 cm. There is provided a thin looped string **64** attached at its both ends to the body of the device. This string **64** is used to readily pull out the probe device from the vaginal cavity. Besides, this string can be used as an antenna for wireless communication.

With reference to main axis **X,** the directions denoted **Yi** and **Wi** are called 'transverse' since they are perpendicular to the main axis **X.**

The body of the device comprises a distal end and a proximal end. Here 'distal' and 'proximal' are defined as follows, in reference to the handling of the device: *'proximal'* refers to a portion of the device remaining outside the vagina, and *'distal'* refers to the opposite direction, i.e. the portion which is the most inserted into the vagina cavity. The above mentioned string **64** is attached to the proximal end part **52.**

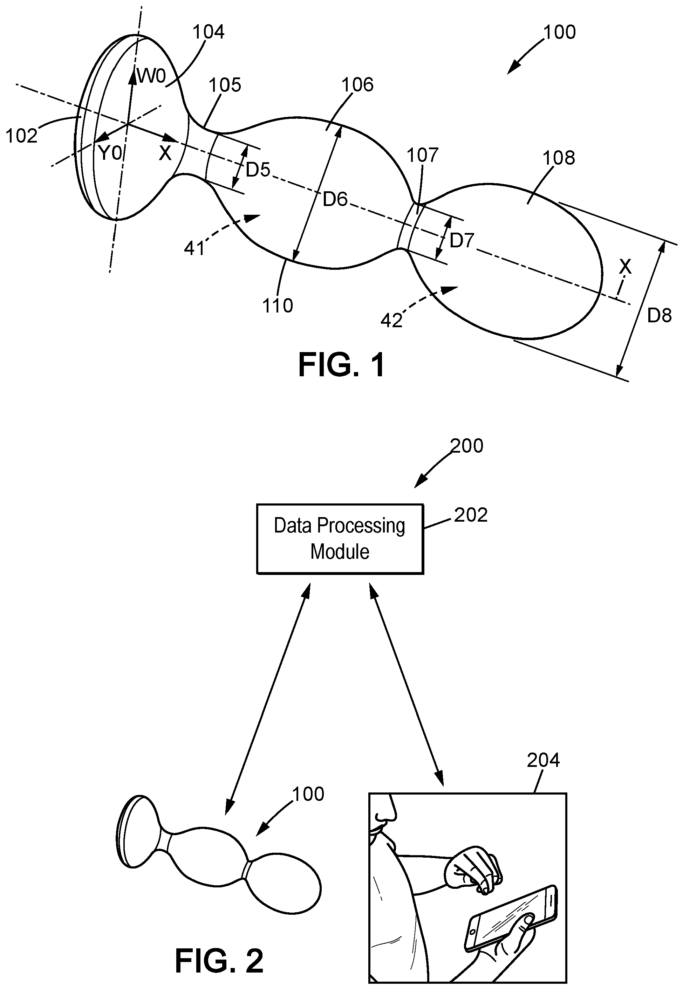

Referring to **FIG. 1,** the pelvic floor probe device **100** is configured to detect harmful and inefficient muscle contractions while performing pelvic floor muscle (PFM) exercises. The device **100** is configured to provide feedback to a user for assisting the user in performing pelvic muscle exercises, thereby strengthening the pelvic floor muscles. The device **100** is further configured to provide data to perform pelvic floor muscle (PFM) exercises to the user or subject.

The device **100** comprises a probe **110** having a first section **106,** a second section **108** and a third section **104.** The second section **108** is a bulbous body that enables the probe **110** to easily penetrate and become inserted within the intended pelvic anatomical passageway, e.g., vagina. As the probe **110** is advanced into the anatomical passageway, the first section **106** is positioned within a midzone area of the pelvic cavity. The second section **108** is positioned within a distal zone of the pelvic cavity. The third section **104** extends from the intermediate first section **106** comprises a proximal end **102** made of at least one material, including, but not limited to, medical-grade silicon. Each section (**108, 106, 104**) of the device **100** is a bulbous shaped body connected to one another to form the probe **110.**

The structure of the probe **110** provides frictional retention radially about the contours of the anatomical passageway such that the device **100** may be captured and anchored against the muscles and tissue surrounding the body opening/pelvic cavity. The device **100** further comprises a sensor assembly including one or more sensors. The one or more sensors comprises a first sensor **41** and a second sensor **42**. The first sensor **41** is disposed within the first section **106** and the second sensor **42** is disposed within the second section **108**. In one embodiment, the first sensor and the second sensor are pressure sensors. In another embodiment, the first sensor and the second sensor are force sensors. The first sensor **41** is placed inside the superficial pelvic floor area (mid zone) for superficial sensing at an intermediate position and the second sensor **42** is placed inside the deep pelvic floor area (distal zone) for deep sensing.

The sensor assembly may be configured to detect pressure data related to pelvic floor muscle contraction on performing PFM exercise by the user.

With reference to Figure 8 and to the distal end of the device, the second section **108** is centered at a second axial position located at a second distance **D2** from the distal end of the probe body. The second distance being comprised between 2,5 cm and 3,5 cm. **D2** can be preferably around 3 cm.

The first section **106** is centered on a first axial position located at a first distance **D1** from the distal end of said probe body, the first distance being comprised between 6 cm and 8 cm. **D1** can be preferably around 7 cm. Also we note that the first sensor has a sensitivity centered on said first axial position. Similarly, the second sensor has a sensitivity centered on said second axial position. We further note that, thanks to these respective positions and the mechanical decoupling, sensitive areas of first and second sensors do not overlap.

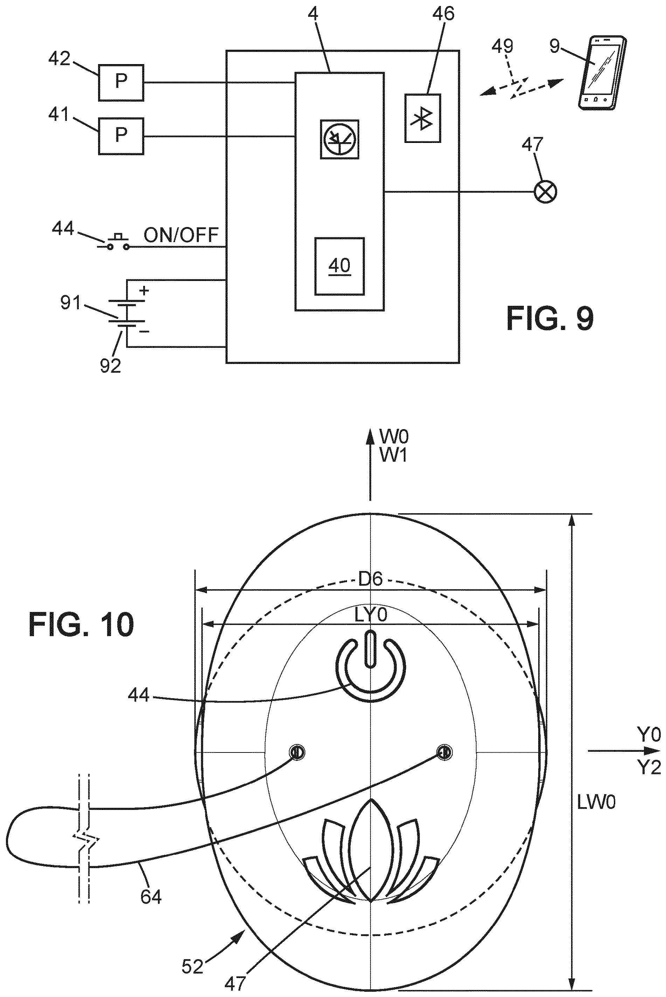

In one embodiment, the first section **106** exhibits a substantially circular cross-section. In this case, the outer diameter denoted **D6** is no greater than 30 mm. Preferably **D6** is about 28 mm or less. Such a small diameter is user-friendly and comfortable. D6 can be seen on figures 1 and 10.

Similarly, the second section **108** exhibits a substantially circular cross section. In this case, the outer diameter denoted **D8** is no greater than 30 mm. Preferably **D8** is about 28 mm or less. Such small diameter is user-friendly and comfortable.

The first section **106** is separated from the second section **108** by a waist section **107**. The waist section has a cross-section (diameter **D7**), which is in size less than 85% of the diameter **D6** of the first section 106. Optionally, the cross-section of the waist section is less than 75% of the cross-section of the first section.

Advantageously, the waist section exhibits flexure compliance to allow a misalignment of the second section **108** relative to the first section **106.**

More precisely, at the waist section **107,** there is arranged between the first section and the second section an assembly flexible element. In the illustrated embodiment, such flexible element is formed as a link ring **6.**

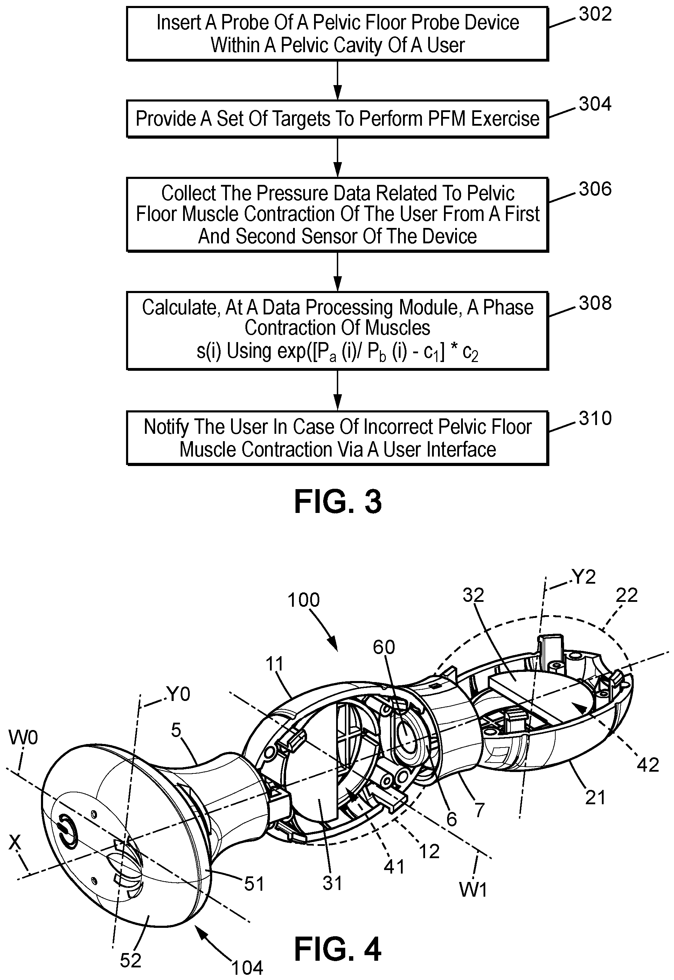

As apparent from figure 4, the link ring arranged at the waist section is encompassed by a **collar 7.** The outer shape of said collar 7 ensures general outward continuity between the two bulbous sections. From a center area (smallest diameter), the collar diverges in both axial directions, i.e. the outside size increases when going away in both opposite directions from the center area.

Further, the third section **104** and the first section are separated from one another by a base waist portion **105.** The base waist section has a cross-section (diameter **D5**) which is in size less than 85% of the diameter **D6** of the first section 106. Optionally, the cross-section of the base waist section is less than 75% of the cross-section of the first section.

Further, as shown at Figure 10, the third section **104** exhibits a transverse section with a length **LW0** and a width **LY0. LW0** is the largest transverse dimension, **LW0** is comprised between 36 mm and 44 mm.

Said otherwise in summary, starting from the base, the device comprises the third section **104,** the base waist portion **105,** the first bulbous section **106,** the waist section **107,** and finally the second bulbous section **108.** The two waist sections **105,107** form substantial restrictions from the cross-section standpoint. The third section **104,** given its respective large cross-section forms an insertion stop. The base waist portion **105** generally prevents the fall of the device, due to perineal elasticity. The waist section **107** provides decoupling of sensing between the first sensor housed in the first section 106 and the second sensor housed in the second section 108.

The above mentioned shape confers to the device an aesthetic and pleasant appearance. Such a wavy device is attractive.

Additionally, the first sensor **41** exhibits a main direction of sensing along a first transverse direction **W1** and the second sensor **42** exhibits a main direction of sensing along a second transverse direction **Y2.**

In practice, for each of the first and second sensors, there may be provided a degree of freedom along its main direction of sensing. Each of the first and second sensors can be variable resistor sensor, in which the mechanical deformation along the main direction of sensing is transduced into a variation of resistance. Said variable resistor can be part of a Wheatstone bridge reading schematics as known per se.

The first sensor **41** is adapted for measuring the pressure applied by the internal vaginal surface to the external surface of the probe at the midzone area of the vaginal canal, and the second sensor **42** is adapted for measuring the pressure applied by the internal vaginal surface to the external surface of the probe at a distal zone. First and second sensors 41,42 operate independently, they have distinct and non-overlapping sensing areas.

Generally, first and second sensors are electronic sensors chosen among electromechanical sensors or pressure sensors or force sensors.

According to one option, the third section **104** has an oblong transverse cross-section. This oblong or oval cross-section has a larger dimension **LW0** along a base orientation noted **W0,** which is parallel to the first transverse direction **W1.** Thanks to this provision, the user is induced to place the device with the base orientation aligned with her anteroposterior direction. The smaller transverse cross-section **LY0** (along Y0) is aligned with the left-right anatomic direction of the user.

We note however that the orientation of the main direction of sensing can be different. For example, the first transverse direction (first sensor) can be orthogonal to the base orientation **W0.** It is not excluded though to have first transverse direction in any oblique direction between WO and Y0, wherein the second transverse direction (second sensor) still orthogonal to the first transverse direction.

Back to the illustrated example, the second transverse direction **Y2** is substantially perpendicular to the first transverse direction **W1.**

Also, both first and second transverse directions (W1,Y2) are substantially perpendicular to the main axis **X.**

The device comprises a fluid-tight envelope **8,** said envelope being made of a biocompatible elastomer. In one embodiment, medical-grade silicon can be chosen. The fluid-tight envelope delimits continuously an internal area of the probe device, whereby no liquid can enter into said internal area.

The fluid-tight envelope **8** is at an external surface of the device, possibly integrally or with the exception of the base end **52** and assembly ring **51.**

A linking member **5** is arranged at the base waist section. The outer shape of said linking member **5** ensures general outer continuity between the first bulbous sections and the third proximal section **104.**

From a center area (smallest diameter), the collar **5** diverges in both axial directions, i.e. the outside size increases when going away in both opposite directions from the center area.

The base end **52** comprises an On/Off button **44,** a LED **47** and one or more coupling for the looped string **64.** The assembly ring **51** is adjacent to the base end **52.** The assembly ring **51** exhibits a small dimension in the axial direction. In the illustrated example. The assembly ring 51 locks an inward return of the proximal border.

According to one option, the pelvic floor probe device comprises a first battery cell **91** and a first battery housing **31** lodged in the first section **106** and a second battery cell and a second battery housing **32** lodged in the second section **108.**

According to one option, the first section comprises two complementary first shells **(11,12),** which, when assembled, form the bulbous body, with a first assembly joint arranged generally on a plane perpendicular to the first transverse direction **W1.** There may be provided a gap/play denoted **GS** (cf Fig 7) along W1 to allow compression of the first section; said otherwise, complementary first shells may be biased away from one another with a small gap/play, and pressure exerted by the anatomic tissue surrounding the complementary first shells move the shells toward one another, tending to decrease the gap. In alternate embodiments, the shells themselves are elastically deformable along **W1** (i.e. perpendicularly of the assembly plane).

The assembly of the first shells may comprise hooks and co-operating recesses. As illustrated, the one of the first shell **11** comprises hooks **84,** in the opposed shell **12,** there provided cooperating recesses **85.** In alternate configurations, it can also be snap fitting means or the like. There may be provided guiding holes **87** and counterpart pins.

The first shell **11** (shown in Fig 4) covers generally half of the first section, except at its longitudinal ends where openings leave a passage for electrical wires. The other first shell **12** (not shown in Fig 4) covers generally the other complementary half of the first section, except at its longitudinal ends where openings are provided.

Also, the second section **108** comprises two complementary second shells **(21,22),** which, when assembled, form the bulbous body, with a second assembly joint arranged generally on a plane perpendicular to the second transverse direction **Y2.** The second shells **21,22** are in the example shown here similar to the first shells described here above.

The assembly and related features of the second shells **21,22** are similar to the assembly of the first shells. The opening at the distal end is optional, it can be present or not.

Broadly speaking, second shell assembly can be similar than the first shell assembly with a 90° rotation about main axis X.

The gap/play **GS** in each of the shell assembly allows from a small range translation movement along the main direction of sensing for each sensor (W1 and Y2 respectively).

Off-axis flexion is allowed by each of the waist sections **105,107.** Flexure movements are independent from one another. According to an exemplary embodiment, an Off-axis flexion of about 15 degrees is provided.

According to one aspect, the link ring **6** comprises an axial passage **60** for electrical wires. The link ring and the wires form a flexible junction for the distal sensor (second sensor).

Since the opening 60 is provided at the waist section, its size is small, let's say 1 cm$^2$ or less. Electrical wires for sensor data and power supply go through the free passage.

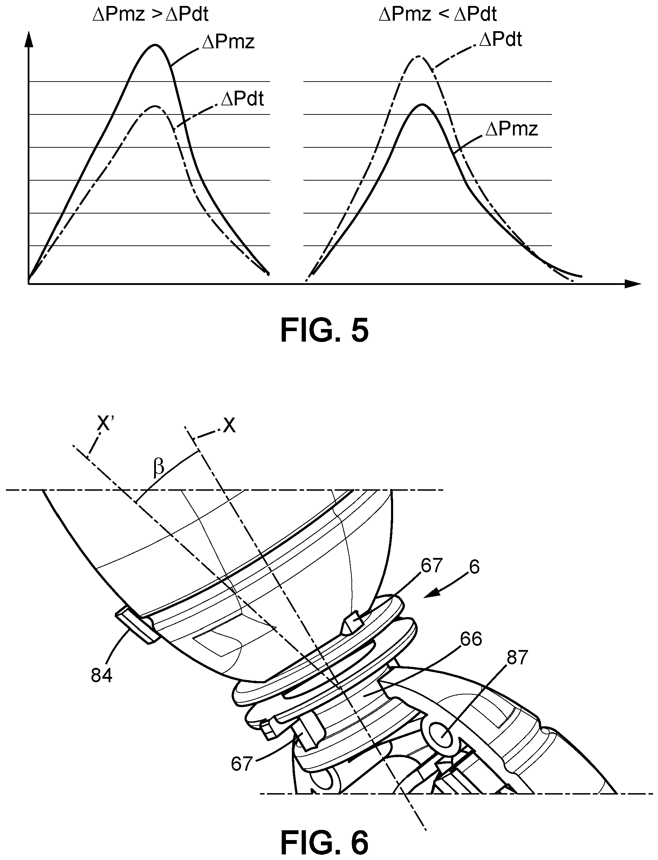

As shown at figure 6, angle denoted β represents the misalignment of axis of the second section with regard to the first section. The elastic mount between first and second sections allows misalignment but provides a rest position corresponding to misalignment β = 0.

According to an exemplary embodiment, nearly no rotation is allowed about axis **X.**

There may be provided lugs **67** in the link ring **6,** said lug being received in a complementary notch arranged at one shell or both shells. A peripheral groove denoted **66** are configured to receive a hemi-circular bead provided at the shell longitudinal border.

A similar or identical flexible joint can also be provided at the base waist section 105. Indeed, here also an Off-axis flexion is permitted, even with a larger angular range, up to 20 degrees in any transverse direction.

Control circuit

A control unit is arranged at the third section. More particularly, a PCB extends transversely to the main axis X and is housed in the third section.

As illustrated at figure 9, the control unit comprises a processor **4,** e.g. a low consumption micro-controller. The control unit comprises a Bluetooth™ coupler **46** allowing for wireless communication link **49.** The wireless communication can be established with a Smartphone **9** or any wireless enabled device. The control unit comprises a Led driver to control the activation of a Led **47,** arranged behind the base end **52.** There is also provided an ON/OFF switch **44.** The switch can be a push button as known per se.

The processor can proceed to auto-power off after a certain time of inactivity at the probe device. The control unit comprises a memory **40,** integrated or not in the micro-controller 4. The processor is connected to the first and second sensors, preferably via an analog-to-digital converters. Digitization can be performed upfront or downstream one or more analog filters.

Signal conditioning applied to sensor signals can take any desired form. Part or all of the signal processing can be done locally or remotely. In other words, what is construed as data processing module can be performed locally within the device. But in some embodiments, part or all of the data processing module can be done remotely, and such case digitized raw signals are wirelessly transmitted. Digital filtering or compression algorithm can be applied prior to transmission.

Use of sensed signals and data

The promoted system comprises a data processing module 2**02** to process the signal provided by first and second sensors. We note that the data processing module **202** can be incorporated in a remote computing device **9** or server. We note that the data processing module **202** can be incorporated in the probe device **100.** The probe device **100** can comprise only signal conditioning. In an alternate embodiment, the probe device can comprise digitization and computing. The computing functional distribution between the probe device **100** and the computing device **9** can be chosen in different manners. The data processing module **202** as described below can be totally or partially comprised in the probe device.

The data processing module **202** is configured to determine a first differential vaginal pressure in the midzone area of the pelvic floor cavity denoted $\Delta\mathbf{Pmz}$ and a second differential pressure in the distal zone area denoted $\Delta\mathbf{Pdt}$. Both first differential pressure $\Delta Pmz$ and second differential pressure $\Delta Pdt$ have as reference pressure values sensed in a relaxed state of the user.

The differential pressures $\Delta Pmz$ and $\Delta Pdt$ are measured relative to the pressure of the PFM in a relaxed state, the reference pressure values are determined by instructing the user to remain in a relaxed state and by measuring the pressures of the midzone and distal zone of the PFC respectively.

It is to be noted that pressures measured as reference are not necessarily identical to measurements delivered by the sensors when the probe device is not inserted inside an anatomic cavity.

As shown at Figure 5, a healthy PFM contraction involves a midzone pressure substantially higher than a pressure in the distal (representative of an intra-abdominal pressure). For example, the left part of Figure 5 shows a time chart where $\Delta Pmz > \Delta Pdt$. Conversely, an unhealthy PFM contraction involves an intra-abdominal pressure higher than expected, notably in a worst case greater than a midzone pressure. For example, the right part of Figure 5 shows a time chart where $\Delta Pmz < \Delta Pdt$.

When the user exercises PFM contraction, the data processing module calculates a ratio $\Delta Pmz/\Delta Pdt$ and compares it to a threshold value denoted **c1.** This threshold value c1 can be given at the start of use of the product. In one embodiment, this threshold value **c1** can be updated after a learning or calibration procedure that will be described below.

As seen above, the data processing module is in communication with a user device, e.g. smartphone **9.** An application is caused to run on the user device, and a display can notify various messages to the user. In particular, in the case the ratio $\Delta Pmz/\Delta Pdt$ is lower than said first threshold value **c1,** a message of suboptimal pelvic floor muscle contraction is given. In an alternate embodiment, a game behavior is changed according to the value of ratio $\Delta\mathbf{Pmz}/\Delta\mathbf{Pdt}$ versus **c1.**

In one embodiment, the data processing module (202) is configured to register a set of differential pressures data $\Delta Pmz(k)$ and $\Delta Pdt(k)$ of sample points (k) during a voluntary pelvic floor muscle contraction (healthy contractions), wherein said data processing module is configured to calculate a result ratio function **RRF.** This result ratio function is defined by RRF (k) = Func ($\Delta$Pmz (k) / $\Delta$Pdt (k)), where RFF(k) is a series of numbers, each number being representative of a quality of the pelvic floor muscle contraction. The function Func can comprise linear or non-linear components, it can include polynomial expressions, normalization feature, time averaging by including older values, etc....

The series of numbers outputted by the result ratio function can be used as an input for a game to be played on a smartphone or tablet. For example, a widget can jump any time a good contraction is determined (e.g. corresponding to a range of values of RRF).

According to one aspect, said result ratio function RRF can be rendered specific to a particular user. For example, in connection with the game, it is possible to adjust some parameters of the RFF functions or parameters of the game response.

Calibration / parametrization

Instead of having c1 determined once and for all, parameter c1 can be made specific to a particular user. For calibration (otherwise called 'parametrization' or 'personalization'), a learning phase id performed. In practice, a user of a particular probe device is caused to exercise maneuvers on the one hand with intra-abdominal pressure, and on the other hand, maneuver without intra-abdominal pressure, i.e. healthy maneuvers for PFM recovery or strengthening.

As per notation, maneuvers with intra-abdominal pressure are marked with $\delta$i=0, and healthy maneuvers for PFM recovery i.e. maneuvers with low intra-abdominal pressure are marked with $\delta$i=1.

The data processing module process the registered set of differential pressures data into a first characterizing function **CF1**= $\Sigma_k$($\Delta$Pmz (k) / $\Delta$Pdt (k) - c1x), wherein an optimal value **c1xop** of **c1x** is chosen so that CF1is positive for $\delta$i=0, and that CF1is negative for $\delta$i=1, wherein the threshold value (c1) being loaded by said optimal value **c1xop** of c1x.

The resulting value c1xop of this optimization calculus is caused to replace a previous value of c1. Therefore, we provide a personalization which is resulting from this learning phase.

According to a further option, c1x can be chosen so that for each indices k and j $\frac{\Delta\text{Pmz(k)}}{\Delta\text{Pdt (k)}} - c1x$ is positive, for

each k where k are indices for which $\delta$i=0, and $\frac{\Delta\text{Pmz(j)}}{\Delta\text{Pdt (j)}} - c1x$ is negative, for each j where j are indices for which $\delta$i=1.

Thus, the optimal value c1xop is a personalized threshold, caused to replace a previous value of c1. Otherwise said, c1xop is chosen to distinguish 'good' contractions with regard to 'bad' contractions for a particular user of interest, i.e. the current user of the probe device.

According to another optional aspect, we defined an optimization function MF to find out the best c1x. MF is defined as MF = $\Sigma_k$($\Delta$Pmz (k) / $\Delta$Pdt (k) - c1x) - $\Sigma_j$($\Delta$Pmz (j) / $\Delta$Pdt (j) - c1x) where k are indices for which $\delta$i=0 and j are indices for which $\delta$i=1.

In this option c1x is chosen so that the function MF is maximized, i.e. a calculus identifies a positive apex which corresponds to a best value **c1xop for c1x.**

In a further option, a second parameter c2 is defined, besides c1.

In this logic, the data processing module is further configured to process a registered set of differential pressures data into a second characterizing function CF2 defined as follows: $\text{CF2} = \frac{1}{1 + \text{EXP}\left[c2x(\Delta\text{Pmz(k)} / \Delta\text{Pdt(k)} - c1x)\right]}$ ('EXP' is the exponential function).

And a cost optimization algorithm is defined according to the following cost function: $Cost(c_{1x}, c_{2x}) = \|CF2 - \delta i\|$ wherein the function $\|*\|$ is a metric distance function.

The calculation identifies **c1** and **c2** for which the cost function **CF2** is minimal, e.g. a negative apex is searched for. At this minimal point, **c1** and **c2** values are recorded and stored. Further those values **c1** and **c2** are used to update previously known **c1** and **c2.**

The data processing module is configured to store the optimal parameters **c1** and **c2** as obtained from the optimization algorithm.

A notification of an occurrence suboptimal contraction is given when $\text{CF2(j)} = \frac{1}{1 + \text{EXP}\left[c2(\Delta\text{Pmz(j)} / \Delta\text{Pdt(j)} - c1)\right]}$ is above 0.5.

The data processing module **202** is in communication with the first and the second groups of sensors. The data processing module is configured to calculate at a given time the differential pressure of the PFM in the midzone of the Pelvic Floor Cavity (PFC) denoted $\Delta$Pmz, and the differential pressure of the PFM in the distal zone of the PFC, denoted $\Delta$Pdt.

The device is configured to determine Valsalva contractions. If the phase contraction is less than zero, the data processing module determines that the pelvic floor muscle contraction is incorrect. Alternatively, if the ratio $\Delta$Pmz /$\Delta$Pdt is lower than a calculated threshold value c1, the processing module determines that the pelvic floor muscle contraction is incorrect.

The user interface is configured to display the data related to incorrect pelvic floor muscle contraction to the user. The data processing module is in communication with a user device to notify the data related to incorrect pelvic floor muscle contraction. The user device is at least one of a desktop computer, laptop computer, tablet, mobile phone, smartphone, or other suitable handheld electronic devices.

According to the present invention, the method and/or system of evaluating pelvic floor muscle (PFM) exercise performed by a user comprise the step of positioning the pelvic floor probe device inserted within a pelvic cavity. The probe comprises one or more sections including, a first section and a second section. The first section and the second section are positioned in contact with tissues of the pelvic cavity. The first section which comprises a group of first sensors is positioned within a midzone area of the pelvic cavity, and the second section has a second group of sensors, positioned within a distal zone of the pelvic cavity. At another step, a set of contraction targets are provided to the subject. The user is prompted to reach the contraction targets by contracting her pelvic floor. For example, the targets may include, but not limited to, contraction start and end flags. The pressure data related to pelvic muscle contraction on performing PFM exercise by the subject is detected using the first group of sensors and the second group of sensors. The detected pressure data of the subject are then collected from the first sensor and the second sensor. The phase contraction of muscles is then calculated according to the algorithm described in the embodiments based on the measures of the mean pressure of contraction numbers i for the first sensor and second sensor. If the phase contraction is less than zero, the data processing module determines that the pelvic floor muscle contraction is incorrect. If the phase contraction is greater than one, the data processing module determines that the pelvic floor muscle contraction is correct. Finally, the incorrect pelvic floor muscle contraction is notified to the subject via the interface of the probe device, which is in communication with the data processing module.

According to a first embodiment, the present invention is thus directed to a pelvic floor muscle (PFM) contraction monitoring system of a subject, comprising:

- a pelvic floor probe device configured to be positioned within the vaginal canal and in contact with the internal vaginal surface of the subject, said probe comprising a body with a distal end and proximal end, an external surface, and at least two sections, the first section (midzone section) being at the proximal end of the probe for contacting the midzone area of the vaginal canal and the second one being positioned at the distal end of the probe for contacting the distal zone of the vaginal canal, each of said sections comprising an array of sensors adapted for measuring the pressure applied by the internal vaginal surface to the external surface of the probe;

- a data processing module configured to receive and process the differential vaginal pressure in the midzone area of the pelvic floor cavity ($\Delta$Pmz) and in the distal zone area ($\Delta$Pdt), to register a set of differential pressures data $\Delta$Pmz $(k_v)$ and $\Delta$Pdt $(k_v)$ of sample points during known (voluntary) Valsalva contractions $(k_v)$, and to register a set of differential pressures data $\Delta$Pmz$(k_h)$ and $\Delta P_{distal}$ $(k_h)$ of sample points during known (voluntary) healthy contractions $(k_h)$, during the internal calibration phase,

- said data processing module being also configured to create two 3-dimensional arrays, a first array based on known Valsalva contraction data: $\{(\Delta$Pmz $(i), \Delta$Pdt $(i), \delta_i)\}$, wherein i are indexes of sampling points registered during a known (voluntary) Valsalva contraction and $\delta_i = 1$; and a second array based on known (voluntary) healthy contraction data: $\{(\Delta$Pmz $(j), \Delta$Pdt $(j), \delta_j)\}$, wherein j are indexes of sampling points registered during a healthy contraction and $\delta_{j=} 0$, and to process the registered set of differential pressures data into a standard cost optimization algorithm according to the following cost function:

$$Cost\,(c1) = \left\|\sigma\left(c1, \Delta\text{Pmz}\,(\text{k}), \Delta\text{Pdt}\,(\text{k})\right) - \delta_k\right\|$$

wherein k represents all sample points either during Valsalva contractions $(k_v)$ or during healthy contractions $((k_h)$, wherein the function $\| * \|$ *is a metric distance function,* and wherein $\sigma(i)$ is calculated as follows:

$$\sigma(i) = \cfrac{1}{1 + e^{\left(\frac{\Delta\text{Pmz(i)}}{\Delta\text{Pdt(i)}} - c1\right)}}$$

- said data processing module being further configured to store the optimal parameter c1 as obtained from the optimization algorithm and to measure the differential pressures $\Delta$Pmz and $\Delta$Pdt of the PFM of a subject during training phase with said arrays of sensors of said at least two sections of the probe and using the data processing module to calculate the ratio ΔPmz / ΔPdt;

- an interface in communication with the data processing module, said interface being configured to receive data from the data processing module that the contraction is an incorrect contraction of the PFM or Valsalva contraction when the ratio is inferior to the threshold value c1, and further configured to notify the subject of the occurrence of a Valsalva contraction.

According to to an example not falling within the scope of the claims, a method of detecting occurrences of incorrect pelvic floor muscle (PFM) contraction or Valsalva contractions of a subject comprising:

- receiving pressure data generated by at least two sections of a pelvic floor probe device, the first section (midzone section) being at the proximal end of the probe device for contacting the midzone area of the vaginal canal of said subject, and the second section (distal zone section) being positioned at the distal end of the probe for contacting the distal zone of the vaginal canal of said subject, each of said sections comprising an array of sensors adapted for measuring the pressure applied by the internal vaginal surface to the external surface of the probe device,
- using a processing module to process the differential vaginal pressure in the midzone area of the pelvic floor cavity (ΔPmz) and in the distal zone area (ΔPdt), to register a dataset of differential pressures $\Delta Pmz_e(k_v)$ and $\Delta Pdt(k_v)$ measured while the subject is performing a set of (voluntary) Valsalva contractions ($k_v$), and to register a dataset of differential pressures $\Delta Pmz(k_h)$ and $\Delta Pdt(k_h)$ measured while the subject is performing a set of known (voluntary) healthy contractions ($k_h$) during the internal calibration phase,
- using the data processing module to create two 3-dimensional arrays, a first array based on Valsalva contraction data: $\{(\Delta Pmz(i), \Delta Pdt(i), \delta_i)\}$, wherein i are indexes of sampling points registered during a Valsalva contraction and $\delta_{i=}1$; and a second array based on healthy contraction data : $\{(\Delta Pmz(j), \Delta Pdt(j), \delta_j)\}$, wherein j are indexes of sampling points registered during a healthy contraction and $\delta_{j=}0$, and
- using the data processing module to process the registered set of differential pressures data into a standard cost optimization algorithm according to the following cost function:

$$Cost\,(c1) = \left\| \sigma\left(c1, \Delta Pmz\,(k), \Delta Pdt\,(k)\right) - \delta_k \right\|$$

wherein k represents all sample points either during Valsalva contractions ($k_v$) or during healthy contractions (($k_h$), wherein the function || * || *is a metric distance function,* and
wherein σ(i) is the sigmoid function calculated as follows:

$$\sigma(i) = \frac{1}{1 + e^{\left(\frac{\Delta Pmz(i)}{\Delta Pdt(i)} - c1\right)}}$$

- using the data processing module to store the optimal parameter c1 as obtained from the optimization algorithm;
- measuring the differential pressures ΔPmz and ΔPdt of the PFM of a subject during the training phase with said arrays of sensors of said at least two sections of the probe and using the data processing module to calculate the ratio ΔPmz / ΔPdt;
- detecting that the contraction is an incorrect contraction of the PFM when the ratio is inferior to the threshold value c1 as calculated above by the data processing module.

According to this first embodiment, the present invention further relates to a pelvic floor probe device for use in detecting incorrect contractions of the pelvic floor muscle (PFM) or Valsalva contractions of a subject, comprising a body with an external surface, a distal end and proximal end, configured to be positioned within the vaginal canal and in contact with the internal vaginal surface of the subject, at least two sections contained within the body of the probe, the first section (midzone section) being at the proximal end of the probe for contacting the midzone area of the vaginal canal and the second section (distal zone section) being positioned at the distal end of the probe for contacting the distal zone of the vaginal canal, each of said sections comprising an array of sensors adapted for measuring the pressure applied by the internal vaginal surface to the external surface of the probe; a data processing module which is contained in the body of the probe and is configured to receive and to process data generated by the sensors as described above; as well as an interface which is configured to receive data generated by the data processing module and to notify the subject that the contraction is an incorrect contraction of the PFM when the ratio is inferior to the threshold calculated value c1.

According to an example not falling within the scope of the claims, a computer device for use in detecting incorrect contractions of the pelvic floor muscle (PFM) or Valsalva contractions of a subject, comprising:

- a receiver configured to receive the threshold value c1 from a pelvic floor probe device;
- a data processing module configured to receive and to process data generated by the sensors as described above; as well as an interface which is configured to receive data generated by the data processing module and to notify the subject that the contraction is an incorrect contraction of the PFM when the ratio is inferior to the threshold calculated value c1 as described above in this embodiment.

According to a second embodiment, the present invention relates to a system and device for detecting incorrect PFM contractions in a subject, wherein the threshold value c1 has been pre-set as a standardized value.

According to this second embodiment, the present invention thus relates to a system and a method of evaluating pelvic floor muscle (PFM) exercises performed by a user, comprising a pelvic floor probe (PFP) comprising two or more sections. Each section is filled with a group of electronic sensors that are measuring the pressure applied by the internal vaginal surface on the surface of the PFP; a data processing module in communication with said sensors, and a user interface in communication with the data processing module, informing the user when they should contract and relax PFM

According to this second embodiment, one group of sensors may be positioned within a midzone area of the pelvic cavity and the second group of sensors may be positioned within a distal zone of the pelvic cavity. The data processing module may be configured to calculate at a given time the differential vaginal pressure in the midzone of the Pelvic Floor Cavity (PFC): $\Delta Pmz$, and the differential vaginal pressure in the distal zone of the PFC: $\Delta Pdt$.

The differential pressures $\Delta Pmz$ and $\Delta Pdt$ may be then measured relative to the pressure of the PFM in a relaxed state, which may be determined by instructing the user to remain in a relaxed state and by measuring the pressures of the midzone and distal zone of the PFC respectively.

The data processing module may be then configured to calculate the ratio $\Delta Pmz / \Delta Pdt$ for each sampling point i and compare it to a threshold value c1. The contraction may be thus evaluated as being potentially incorrect if the ratio $\Delta Pmz / \Delta Pdt$ is lower than the threshold value c1.

According to this embodiment, the coefficients c1 may be calculated using the following steps. As a first initialization step, c1 may be assigned a fixed pre-set value within an interval comprised between 0.8 and 4, or between 1 and 3. The threshold value c1 is preferably preset between 1 and 2, and most preferably c1 is equal to 1.2.

The data processing module is thus configured to measure the differential pressures $\Delta Pmz$ and $\Delta Pdt$ of the PFM of a subject during the training phase with said arrays of sensors of said at least two sections of the probe and using the data processing module to calculate the ratio $\Delta Pmz / \Delta Pdt$; and to detect and notify said subject that the contraction is an incorrect contraction of the PFM when the ratio is inferior to the threshold pre-set value c1.

According to this second embodiment, the present invention also relates to a pelvic floor probe device comprising a probe adapted to position within a pelvic cavity of a user. The probe device itself comprises a first section comprising a group of sensors which is positioned within a midzone area of the pelvic cavity and a second section comprising a group of sensors which is positioned within a distal zone of the pelvic cavity. The first group of sensors and the second group of sensors are configured to detect the pressure applied on the probe surface by the internal surface of the vagina.

The probe device also comprises a data processing module in communication with the first and second groups of sensors. The data processing module is configured to calculate at a given time the differential pressure of the PFM in the midzone of the Pelvic Floor Cavity (PFC) referred as $\Delta Pmz$, and the differential pressure of the PFM in the distal zone of the PFC, referred as $\Delta Pdt$.

As described above, the differential pressures $\Delta Pmz$ and $\Delta Pdt$ may be measured relative to the pressure of the PFM in a relaxed state. The subject may be for example instructed to remain in a relaxed state during which the pressures of the midzone and distal zone of the PFC, respectively, may be measured. The data processing module may then determine whether the pelvic floor muscle contraction is incorrect, if the ratio $\Delta Pmz / \Delta Pdt$ is lower than the threshold pre-set value c1 as described above.

The system further comprises a display in communication with the data processing module, which is configured to display a set of contraction targets that the subject is prompted to reach by contracting her pelvic floor. Preferably, the targets include contraction start and end flags, and displays data related to incorrect pelvic floor muscle contraction to the subject.

According to another embodiment, the present invention relates to a system and device as described in the above embodiment, with the difference that the data processing module may be configured to calculate a value q of the quality of the pelvic floor muscle contraction which reflects parasite or incorrect contractions of the subject at each sampling point (i), that is a contraction during which the intra-abdominal pressure is superior to the pelvic pressure.

The value q(i) at each sampling point is calculated by the data processing module with the following function:

$$q(i) = e^{c2*(\frac{\Delta Pmidzone(i)}{\Delta Pdistal(i)} - c1)} .$$

The coefficients c1 and c2 may be calculated using the following steps: as a first initialization step, the coefficient c1 is assigned a fixed value within an interval comprised between 0.8 and 4, and the coefficient c2 is assigned a fixed value comprised within the interval comprised between of -20 and +20. Preferably, c1 is assigned a fixed value between 1 and 2 and c2 is assigned a value between -10 and +10. Most preferably, c1 is assigned a value equal to 1.2 and c2 is assigned a value equal to 2.

The subject or user is then prompted by the interface of the device to Valsalva maneuver (after providing a clear explanation on how to perform it). While the user performs the Valsalva maneuvre, the data processing module registers a set of experimental differential pressures $\Delta Pmz(k_v)$ and $\Delta Pdt(k_v)$. The user or subject is subsequently prompted to a healthy pelvic floor contraction (after providing a clear explanation on how to perform it). While the user performs a regular pelvic floor contraction the data processing module registers a set of experimental differential pressures $\Delta Pmz(k_h)$ and $\Delta Pdt(k_h)$.

The data processing module creates two 3-dimensional arrays with experimental Valsalva contraction training data: $\{(\Delta Pmz(i), \Delta Pdt(i), \delta_i)\}$ such as i are indexes of a sampling points are registered during a Valsalva contraction and $\delta_{i=}$ 1; and with experimental Healthy contraction training data: $\{(\Delta Pmz(j), \Delta Pdt(j), \delta_j)\}$ such as j are indexes of sampling points registered during a healthy contraction and $\delta_{j=}$ 0.

The data processing unit uses a standard cost optimization algorithm with a cost function $Cost(c1, c2) = \|q^*(c_1, c_2, \Delta P_{mz}(k), \Delta P_{dt}(k)) - \delta_k\|$ *over all sample points k where* $\| * \|$ *is a metric distance function.* Such cost optimization algorithm outputs the optimal parameters $c_1^*$ and $c_2^*$ that are stored by the data processing module and are used as parameters for calculating q and q* numbers.

The data processing module is thus further configured to detect and generate output information to the interface that an incorrect PFM contraction has been performed when the intra-abdominal pressure is superior to the pelvic pressure.

Preferably, the data processing module is used to process a rectified value $q^*(i) = \frac{1}{1+a(i)}$ which takes values in $[0,1]$, and is used to detect and output information of an incorrect PFM contraction when q*(i) >0,5.

According to the above embodiments of the present invention, cost optimization algorithms may be chosen among gradient descent, stochastic gradient descent, and Newton's method.

Also, said first and second sections of the probe device may be positioned in contact with tissues of the pelvic cavity at a relative distance of approximately 3 cm and 7 cm, respectively, from the distal end of the said probe body.

The pelvic floor probe device may further comprise a third section at the distal end of the body of the probe device.

The sections of the probe may be of any suitable shape and preferably have a round smooth shape and most preferably a bulbous shape. The sections of the probe device may be made of any suitable medical material. Preferably, they are made of medical-grade silicone.

Furthermore, the sensors which are included in probe device sections may comprise electronic sensors chosen among pressure sensors or force sensors.

A description of embodiments of the present invention will now be given with reference to the Figures. It is expected that the present invention may be embodied in other specific forms without departing from the scope of the appended claims. The described embodiments are to be considered in all respects only as illustrative and not restrictive.

Referring to **FIG. 2,** an environment **200,** where the pelvic floor probe device having a data processing module **202** in communication with a user device is disclosed. The data processing module **202** is in communication with the first and second sensor. In another embodiment, the device **100** further comprises a display in communication with the data processing module **202.** The data processing module **202** to collect, store and process the data from the sensor assembly. In one embodiment, data processing module **202** comprises a set of program instructions or algorithms. The data processing module **202** is configured to provide a set of targets to perform the PFM exercises. The set of targets are displayed at the display. The data processing module **202** is configured to calculate phase contraction of muscles s(i) using $\exp([pa(i)/pb(i) - c1] * c2)$, wherein c1 and c2 are defined constants, wherein Pa(i) and Pb(i) represents mean pressure of contraction number i for first sensor and second sensor, respectively. Various calibration methods could be used to initialize the values of constants c1 and c2. The data processing module **202** is configured to detect Valsalva contractions.

The data processing module **202** is configured to determine the pelvic floor muscle contraction as incorrect, if the phase contraction is less than zero. The data processing module **202** is configured to determine the pelvic floor muscle contraction as correct, if the phase contraction is greater than one. The data related to incorrect pelvic floor muscle contraction/ feedback related to pelvic floor muscle contraction is displayed to the user, via the display. In one embodiment, the feedback is notified at a user device associated with the user. In another embodiment, the feedback is notified at the user interface

As described above, the device may be chosen among a desktop computer, laptop computer, tablet, mobile phone, smartphone, or handheld electronic devices. In one embodiment, the data processing module 2**02** is incorporated in a remote computing device or server, which is in communication with the sensor assembly via a wireless network. In another embodiment, the data processing module **202** is incorporated in the user device, which is in communication with the sensor assembly via the wireless network. In one embodiment, the network could be, but not limited to, Wi-Fi, WiMAX, Bluetooth®, and wireless local area network (WLAN). In some embodiments, the computing device could be a touchscreen and/or non-touchscreen and adapted to run on any type of OS, such as iOS™, Windows™, Android™, Unix™, Linux™ and/or others. In one embodiment, the server is at least one of a general or special purpose computer. The computing device could be operated as a single computer, which can be a hardware and/or software server, a workstation, a mainframe, a supercomputer, a server farm, and so forth.

Referring to **FIG. 3,** an example not falling within the scope of the claims, provides a method **300** of evaluating pelvic floor muscle (PFM) exercise performed by the user. At step **302,** the probe **110** of the pelvic floor probe device **100** is inserted within the pelvic cavity of the user. At step **304,** a set of targets to perform PFM exercise is provided to the user. At step **306,** the pressure data related to the pelvic floor muscle contraction is collected from the sensor assembly. At step **308,** the data processing module **202** calculates phase contraction of muscles as described in the above embodiments. The data processing module **202** determines the pelvic floor muscle contraction as incorrect, if the phase contraction is less than zero. The data processing module **202** determines the pelvic floor muscle contraction as correct, if the phase contraction is greater than one. At step **310,** the user is notified in case of incorrect pelvic floor muscle contraction at the user interface.

According to the example, the method allows detecting harmful and/or inefficient muscle contractions during women pelvic floor rehabilitation exercises (called Kegel exercises) or any other kind of PFM exercises using the internal pelvic floor probe device **100** equipped with force or pressure sensors. The method, particularly, allows to detect Valsalva contractions. The method of the example is implemented via the device **100** with two pressure or force sensors, provided these are placed in the midzone and distal zone of the user's vagina. This makes the monitoring of incorrect PFM in a cheaper, faster and more practical way than other existing methods.

Miscellaneous

There is no liquid fluid contained in the device. The device houses captive air, but it is noted that the sensors do not measure the pressure of this captive air.

The bulbous shape may have a slightly oval cross-section, i.e. with a size larger in the sense direction than in the orthogonal direction. In another embodiment, the slightly oval cross section exhibits a smaller size in the sense direction than a size in the orthogonal direction.

The assembly ring 51 can be made of light alloy or hard plastics. The shells are made of plastics like polyethylene or the like.

**Claims**

1.  A **system** for monitoring pelvic floor muscle (PFM) contractions performed by a subject, notably a female individual, the system comprising:

    - a **pelvic floor probe device** (100) configured to be positioned within the vaginal canal and in contact with the internal vaginal surface of the subject, said probe comprising a **body** with a **distal end** and **proximal end,** an external surface, and at least **two sections,** a **first section** (106) being at the proximal end of the probe for contacting the midzone area of the vaginal canal and a **second section** (108) being positioned at the distal end of the probe for contacting the distal zone of the vaginal canal, each of said first and second sections having a **bulbous shape,** the first section comprising at least a **first sensor** adapted for measuring the pressure applied by the internal vaginal surface to the external surface of the probe at the midzone area of the vaginal canal, and the second section comprising at least a **second sensor** adapted for measuring the pressure applied by the internal vaginal surface to the external surface of the probe at a distal zone,

    - a **data processing module** (202) configured to determine a **first differential vaginal pressure** in the midzone area of the pelvic floor cavity denoted $\Delta Pmz$ and a **second differential pressure** in the distal zone area denoted $\Delta Pdt$, first and a second differential pressures having as reference pressure values sensed in a **relaxed** state of the user,

    and the data processing module (202) is configured to calculate at least one ratio $\Delta Pmz/\Delta Pdt$ and compare it to a **threshold value** ($c_1$),

wherein the data processing module is in communication with a **user device** to notify, in the case the ratio is lower than said first threshold value, a suboptimal pelvic floor muscle contraction.

2. The system of claim 1, wherein the pelvic floor probe device (100) extends along a **main axis** (X), wherein the **first sensor** exhibits a main direction of sensing along a first transverse direction (W1) and the **second sensor** exhibits a main direction of sensing along a second transverse direction (Y2), wherein said second transverse direction is **substantially perpendicular** to the first transverse direction, and preferably, first and second transverse directions (W1,Y2) are substantially perpendicular to the main axis (X).

3. The system according to any of claims 1 to 2, wherein said first section (106) is separated from said second section (108) by a **waist section** (107) said waist section having a cross-section (D7) in size less than 75% of the size of the cross-section (D6) of said first section (106) and wherein the waist section exhibits **flexure** compliance to allow a misalignment of said second section (108) relative to the said first section (106).

4. The system according to any of claims 1 to 3, wherein the pelvic floor probe device (100) comprises a **third section** (104) arranged at the proximal end of the device, and the third section (104) is separated from the first section (106) by a **base waist portion** (105).

5. The system of claim 2, wherein the **third section** (104) has an **oblong** transverse cross-section with a larger dimension along a base orientation noted W0, which is parallel to the first transverse direction (W1), whereby the user is induced to place the device with the base orientation aligned with her anteroposterior direction.

6. The system according to any of claims 1 to 5, wherein the pelvic floor probe device (100) comprises a **first battery cell** lodged in the first section (106) and a **second battery cell** lodged in the second section (108).

7. The system according to any of claims 3 to 6, wherein first section (106) and second section (108) are attached together by a **link ring** (6) arranged at the **waist section** (107), the **link ring** (6) being encompassed by a **collar** (7).

8. The system according to any of claims 2 to 5, wherein the first section (106) comprises two **complementary first shells** (11,12), which, when assembled, form the bulbous body, with a first assembly joint arranged generally on a plane perpendicular to the first transverse direction W1.

9. The system according to any of claims 2 to 5, wherein the second section (108) comprises two **complementary second shells** (21,22), which, when assembled, form the bulbous body, with a second assembly joint arranged generally on a plane perpendicular to the second transverse direction Y2.

10. The system according to any of claims 1 to 9, wherein the first section (106) exhibits a substantially circular cross section (D6), with an **outer diameter** no greater than 30 mm, preferably no greater than 28 mm.

11. The system according to any one of claims 1 to 10, wherein said first and second sensors are electronic sensors chosen among **electromechanical** sensors or **pressure sensors** or **force sensors.**

12. The system according to any one of claims 1 to 11, wherein the first section is centered on a first axial position located at a first distance from the distal end of said probe body, the first distance being comprised **between 6 and 8 cm,** preferably 7cm, and the second section is centered on a second axial position located at a second distance from the distal end of said probe body, the second distance being comprised **between 2,5 and 3,5 cm,** preferably 3cm.

13. The system according to any one of claims 1 to 12, wherein the device comprises a fluid-**tight envelope** (8), said envelope being made of a biocompatible elastomer, preferably a medical grade silicon.

14. The system according to any of claims 1 to 13, wherein the **data processing module** (202) is configured to register a set of differential pressures data $\Delta Pmz(k_v)$ and $\Delta Pdt(k_v)$ of sample points during known (voluntary) Valsalva contractions ($k_v$), and to register a set of differential pressures data $\Delta Pmz(k_h)$ and $\Delta Pdt(k_h)$ of sample points during known (voluntary) healthy contractions ($k_h$), during the internal calibration phase,- said data processing module being also configured to create two multi-dimensional arrays, a first array based on known Valsalva contraction data: $\{(\Delta Pmz (i), \Delta Pdt (i), \delta_i)\}$, wherein i are indexes of sampling points registered during a known (voluntary) Valsalva contraction and $\delta_i = 1$; and a second array based on known (voluntary) healthy contraction data: $\{(\Delta Pmz (j), \Delta Pdt (j), \delta_j)\}$, wherein j are indexes of sampling points registered during a healthy contraction and $\delta_j = 0$, and to process the registered set of differential pressures data into a standard cost optimization algorithm according to the following cost function:

$$Cost\ (c1) = \left\| \sigma \left(c1, \Delta \text{Pmz}\ (k), \Delta \text{Pdt}\ (k)\right) - \delta_k \right\|$$

wherein k represents all sample points either during Valsalva contractions ($k_v$) or during healthy contractions (($k_h$), wherein the function $\| * \|$ *is a metric distance function,* and
wherein $\sigma(i)$ is calculated as follows:

$$\sigma(i) = \frac{1}{1 + e^{\left(\frac{\Delta \text{Pmz(i)}}{\Delta \text{Pdt(i)}} - 1\right)}}$$

- said data processing module being further configured to store the optimal parameter c1 as obtained from the optimization algorithm and to measure the differential pressures Δ**Pmz** and Δ**Pdt** of the PFM of a subject during training phase with said arrays of sensors of said at least two sections of the probe and using the data processing module to calculate the ratio Δ**Pmz** / Δ**Pdt,**
- an interface in communication with the data processing module, said interface being configured to receive data from the data processing module that the contraction is an incorrect contraction of the PFM or Valsalva contraction when the ratio is inferior to the threshold value c1, and further configured to notify the subject of the occurrence of a Valsalva contraction.

15. The system according to claim 14, wherein said data processing module is further configured to process a value q of the quality of the pelvic floor muscle contraction at each sampling point (i) wherein q(i) is calculated as follows:

$$q(i) = e^{c2*\left(\frac{\Delta Pmz(i)}{\Delta Pdt(i)} - c1\right)}$$

wherein c1 is assigned a value comprised between 0.8 and 4, or between 1 and 3, or between 1 and 2, or is equal to 1.2;
wherein c2 is assigned a value comprised between -20 and +20, or between -10 and +10, or between 0 and 5 or equal to 2; and
wherein said data processing module is configured to detect and output information that an incorrect PFM contraction has been performed when the intra-abdominal pressure is superior to the pelvic pressure.

16. The system of claim 15, wherein said data processing module is configured to process a rectified value $q^*(i) = \frac{1}{1+q(i)}$ which takes values in [0,1], and to detect and output information of an incorrect PFM contraction when q*(i) >0,5.

17. The system of any one of claims 14 to 16, further comprising a computer device configured to communicate with the data processing module of the probe device to convey the output generated by the data processing module to the computer device of the subject, wherein the probe device further comprises a transmitter configured to send the output data generated by the data processing module to the computer device.

18. The system according to any of claims 1 to 13, wherein the data processing module (202) is further configured to register a set of differential pressures data ΔPmz(k) and ΔPdt(k) of sample points (k) during a voluntary **pelvic floor muscle contraction,**
wherein said data processing module is configured to calculate a result ratio function RRF defined as RRF (k)= Func (ΔPmz (k) / ΔPdt (k)), where RFF(k) is a series of numbers, each number being representative of a quality of the **pelvic floor muscle contraction.**

19. The system according to any of claims 1 to 13, wherein the data processing module (202) is further configured to register, during a **calibration phase,** a set of differential pressures data ΔPmz(kv) and ΔPdt(kv) of sample points (kv) during voluntary intra-**abdominal contraction maneuvers,** and to register a set of differential pressures data ΔPmz(kh) and ΔPdt(kh) of sample points (kh) during voluntary **pelvic floor muscle contractions,**

- said data processing module being also configured to create two multi-dimensional arrays, a **first array** based on known intra-**abdominal contraction maneuvers** data: {( $\Delta$Pmz (i) , $\Delta$Pdt (i) , $\delta_i$)}, wherein i are indexes of sampling points registered during intra-**abdominal contraction maneuvers** and $\delta$i= 1; and a **second array** based on **pelvic floor muscle** contraction data : {( $\Delta$Pmz (j), $\Delta$Pdt (j), $\delta_j$)}, wherein j are indexes of sampling points registered during a **pelvic floor muscle contractions** and $\delta$j = 0, and to process the registered set of differential pressures data into a first characterizing function **CF1**= $\Sigma_k$($\Delta$Pmz (k) / $\Delta$Pdt (k) - c1x),

wherein an **optimal value c1xop** of **c1x** is chosen so that CF1 is **positive** for $\delta$i=0, and that CF1 is **negative** for $\delta$i=1, wherein the **threshold value** (c1) being loaded by said optimal value **c1xop** of c1x,

- said data processing module being further configured to store the **threshold value** (c1) as obtained from the internal calibration phase.

20. The system of claim 19, wherein said data processing module is further configured to process the registered set of differential pressures data into a second characterizing function CF2 defined as follows:

$$CF2 = \frac{1}{1 + e^{\left(c2\frac{\Delta\mathrm{Pmz(i)}}{\Delta\mathrm{Pdt(i)}} - c1\right)}}$$

And a cost optimization algorithm is defined according to the following cost function:

$$Cost(c_{1x}, c_{2x}) = ||CF2 - \delta i||$$

wherein the function ‖*‖ is a metric distance function and

- said data processing module being further configured to store the optimal parameters c1 and c2 as obtained from the optimization algorithm,
- an interface in communication with the data processing module, said interface being configured to receive data from the data processing module and notify an occurrence of an incorrect contraction when

$$CF2(j) = \frac{1}{1 + \mathrm{EXP}\left[c2(\Delta\mathrm{Pmz(j)} / \Delta\mathrm{Pdt(j)} - c1)\right]} \quad \text{is above 0,5.}$$

**Patentansprüche**

1. System zur Überwachung der Kontraktionen der Beckenbodenmuskulatur (PFM), die von einer Person, insbesondere einer Frau, ausgeführt werden, wobei das System Folgendes umfasst:

- eine Beckenbodensonden-Vorrichtung (100), die so konfiguriert ist, dass sie innerhalb des Vaginalkanals und in Kontakt mit der inneren Vaginaloberfläche der Person positioniert werden kann, wobei diese Sonde einen Körper mit einem distalen Ende und einem proximalen Ende, einer äußeren Oberfläche und mindestens zwei Abschnitten umfasst, so dass sich ein erster Abschnitt (106) am proximalen Ende der Sonde befindet, um den Mittelzonenbereich des Vaginalkanals zu berühren, und ein zweiter Abschnitt (108) am distalen Ende der Sonde positioniert ist, um die distale Zone des Vaginalkanals zu berühren. Jeder dieser beiden Abschnitte weist eine bauchige Form auf, wobei der erste Abschnitt mindestens einen ersten Sensor umfasst, der geeignet ist, den Druck zu messen, der von der inneren Scheidenoberfläche auf die äußere Oberfläche der Sonde im Mittelzonenbereich des Vaginalkanals ausgeübt wird, und der zweite Abschnitt mindestens einen zweiten Sensor umfasst, der geeignet ist, den Druck zu messen, der von der inneren Scheidenoberfläche auf die äußere Oberfläche der Sonde in der distalen Zone ausgeübt wird,
- ein Datenverarbeitungsmodul (202), das so konfiguriert ist, dass es einen ersten vaginalen Differenzdruck im mittleren Bereich der Beckenbodenhöhle mit der Bezeichnung $\Delta$Pmz und einen zweiten Differenzdruck im distalen Bereich mit der Bezeichnung $\Delta$Pdt bestimmt, wobei der erste und der zweite Differenzdruck als Referenzdruckwerte dienen, die in einem entspannten Zustand der Benutzerin erfasst werden,

und das Datenverarbeitungsmodul (202) so konfiguriert ist, dass es mindestens ein Verhältnis $\Delta$Pmz/$\Delta$Pdt berechnet und es mit einem Schwellenwert (c1) vergleicht,

wobei das Datenverarbeitungsmodul mit einem Benutzergerät kommuniziert, um im Falle, dass das Verhältnis niedriger als der genannte erste Schwellenwert ist, eine suboptimale Beckenbodenmuskelkontraktion zu melden.

**2.** Das System nach Anspruch 1, wobei sich die Beckenbodensonden-Vorrichtung (100) entlang einer Hauptachse (X) erstreckt, wobei der erste Sensor eine Hauptabtastrichtung entlang einer ersten Querrichtung (W1) aufweist und der zweite Sensor eine Hauptabtastrichtung entlang einer zweiten Querrichtung (Y2) aufweist, wobei die genannte zweite Querrichtung im Wesentlichen senkrecht zur ersten Querrichtung ist und vorzugsweise die erste und die zweite Querrichtung (W1, Y2) im Wesentlichen senkrecht zur Hauptachse (X) sind.

**3.** Das System nach einem der Ansprüche 1 bis 2, wobei der erste Abschnitt (106) vom zweiten Abschnitt (108) durch einen Taillenabschnitt (107) getrennt ist, wobei der Taillenabschnitt einen Querschnitt (D7) von weniger als 75 % der Größe des Querschnitts (D6) des ersten Abschnitts (106) aufweist und wobei der Taillenabschnitt eine Biegenachgiebigkeit aufweist, um eine abweichende Ausrichtung des zweiten Abschnitts (108) relativ zum ersten Abschnitt (106) zu ermöglichen.

**4.** Das System nach einem der Ansprüche 1 bis 3, wobei die Beckenbodensonden-Vorrichtung (100) einen dritten Abschnitt (104) umfasst, der am proximalen Ende der Vorrichtung angeordnet ist, und der dritte Abschnitt (104) vom ersten Abschnitt (106) durch einen Basis-Taillenabschnitt (105) getrennt ist.

**5.** Das System nach Anspruch 2, wobei der dritte Abschnitt (104) einen länglichen Querschnitt mit einer größeren Dimension entlang einer Basisausrichtung (WO) aufweist, die parallel zur ersten Querrichtung (W1) verläuft, wodurch die Benutzerin veranlasst wird, die Vorrichtung so zu platzieren, dass die Basisausrichtung mit ihrer anteroposterioren Richtung fluchtet.

**6.** Das System nach einem der Ansprüche 1 bis 5, wobei die Beckenbodensonden-Vorrichtung (100) eine erste Batteriezelle, die im ersten Abschnitt (106) untergebracht ist, und eine zweite Batteriezelle, die im zweiten Abschnitt (108) untergebracht ist, umfasst.

**7.** Das System nach einem der Ansprüche 3 bis 6, bei dem der erste Abschnitt (106) und der zweite Abschnitt (108) durch einen am Taillenabschnitt (107) angeordneten Verbindungsring (6) miteinander verbunden sind, wobei der Verbindungsring (6) von einer Manschette (7) umschlossen ist.

**8.** Das System nach einem der Ansprüche 2 bis 5, wobei der erste Abschnitt (106) zwei komplementäre erste Schalen (11, 12) umfasst, die, wenn sie zusammengesetzt sind, den bauchigen Körper bilden, mit einer ersten Montageverbindung, die im Allgemeinen in einer Ebene senkrecht zur ersten Querrichtung W1 angeordnet ist.

**9.** Das System nach einem der Ansprüche 2 bis 5, wobei der zweite Abschnitt (108) zwei komplementäre zweite Schalen (21, 22) umfasst, die, wenn sie zusammengesetzt sind, den bauchigen Körper bilden, mit einer zweiten Montageverbindung, die im Allgemeinen in einer Ebene senkrecht zur zweiten Querrichtung Y2 angeordnet ist.

**10.** Das System nach einem der Ansprüche 1 bis 9, wobei der erste Abschnitt (106) einen im Wesentlichen kreisförmigen Querschnitt (D6) mit einem Außendurchmesser von nicht mehr als 30 mm, vorzugsweise nicht mehr als 28 mm, aufweist.

**11.** Das System nach einem der Ansprüche 1 bis 10, wobei der erste und der zweite Sensor elektronische Sensoren sind, die unter elektromechanischen Sensoren oder Drucksensoren oder Kraftsensoren ausgewählt wurden.

**12.** Das System nach einem der Ansprüche 1 bis 11, wobei der erste Abschnitt an einer ersten axialen Position zentriert ist, die sich in einem ersten Abstand vom distalen Ende des Sondenkörpers befindet, wobei der erste Abstand zwischen 6 und 8 cm, vorzugsweise 7 cm, liegt, und der zweite Abschnitt an einer zweiten axialen Position zentriert ist, die sich in einem zweiten Abstand vom distalen Ende des Sondenkörpers befindet, wobei der zweite Abstand zwischen 2,5 und 3,5 cm, vorzugsweise 3 cm, liegt.

**13.** Das System nach einem der Ansprüche 1 bis 12, wobei die Vorrichtung eine flüssigkeitsdichte Hülle (8) umfasst, die aus einem biokompatiblen Elastomer, vorzugsweise einem medizinischen Silikon, hergestellt wurde.

**14.** Das System nach einem der Ansprüche 1 bis 13, wobei das Datenverarbeitungsmodul (202) so konfiguriert ist, dass es einen Satz von Differenzdruckdaten $\Delta Pmz_{(kv)}$ und $APdt_{(kv)}$ von Probenpunkten während bekannter (willentlicher) Valsalva-Kontraktionen $_{(kv)}$ registriert, und einen Satz von Differenzdruckdaten $\Delta Pmz(kh)$ und $\Delta Pdt(kh)$ von Probenpunkten während bekannter (willentlicher) gesunder Kontraktionen $_{(kh)}$ während der internen Kalibrierungsphase registriert, - wobei das Datenverarbeitungsmodul auch so konfiguriert ist, dass es zwei mehrdimensionale Reihen erzeugt, wobei eine erste Reihe auf bekannten Valsalva-Kontraktionsdaten basiert: $\{(\Delta Pmz(i), \Delta Pdt(i), \delta_i)\}$, wobei i Indizes von Messpunkte sind, die während einer bekannten (willentlichen) Valsalva-Kontraktion registriert wurden, und $\delta i = 1$; und eine zweite Reihe, die auf bekannten (willentlichen) gesunden Kontraktionsdaten basiert: $\{(\Delta Pmz(j), \Delta Pdt(j), \delta j)\}$, wobei j die Indizes der Messpunkte sind, die während einer gesunden Kontraktion registriert wurden, und $\delta j = 0$, und dass der registrierte Differenzdruck-Datensatz in einem Standard-Aufwandsoptimierungsalgorithmus gemäß der folgenden Aufwandsfunktion verarbeitet wird:

$$Cost\,(c1) = \left\| \boldsymbol{\sigma}\left(c1, \Delta\mathrm{Pmz}\,(k), \Delta\mathrm{Pdt}\,(k)\right) - \delta_k \right\|$$

wobei k alle Messpunkte entweder während Valsalva-Kontraktionen (10) oder während gesunder Kontraktionen ($_{(kh)}$) darstellt,
wobei die Funktion $\|*\|$ *eine metrische Abstandsfunktion ist,* und
wobei $\sigma(i)$ wie folgt berechnet wird:

$$\sigma(i) = \frac{1}{1 + e^{\left(\frac{\Delta\mathrm{Pmz(i)}}{\Delta\mathrm{Pdt(i)}} - 1\right)}}$$

- das genannte Datenverarbeitungsmodul ist ferner so konfiguriert, dass es den optimalen Parameter c 1 speichert, wie er vom Optimierungsalgorithmus erhalten wurde, und dass es die Differenzdrücke $\Delta Pmz$ und $\Delta Pdt$ der PFM einer Person während der Trainingsphase mit den genannten Sensorreihen der mindestens zwei Abschnitte der Sonde misst und das Datenverarbeitungsmodul verwendet, um das Verhältnis $\Delta Pmz / \Delta Pdt$ zu berechnen,
- eine Schnittstelle, die mit dem Datenverarbeitungsmodul kommuniziert, wobei die Schnittstelle so konfiguriert ist, dass sie vom Datenverarbeitungsmodul Daten empfängt, dass die Kontraktion eine falsche Kontraktion der PFM oder eine Valsalva-Kontraktion ist, wenn das Verhältnis unter dem Schwellenwert cl liegt, und ferner so konfiguriert ist, dass sie die Person über das Auftreten einer Valsalva-Kontraktion informiert.

**15.** Das System nach Anspruch 14, wobei das Datenverarbeitungsmodul ferner so konfiguriert ist, dass es einen Wert q zur Qualität der Beckenbodenmuskelkontraktion an jedem Messpunkt (i) verarbeitet, wobei q(i) wie folgt berechnet wird:

$$q(i) = e^{c2*\left(\frac{\Delta Pmz(i)}{\Delta Pdt(i)} - c1\right)}$$

wobei cl ein Wert zwischen 0,8 und 4 oder zwischen 1 und 3 oder zwischen 1 und 2 zugewiesen wird oder gleich 1,2 ist;
wobei c2 ein Wert zwischen -20 und +20, oder zwischen -10 und +10, oder zwischen 0 und 5 oder gleich 2 zugewiesen wird; und
wobei das Datenverarbeitungsmodul konfiguriert ist, um Informationen zu erkennen und auszugeben, dass eine falsche PFM-Kontraktion durchgeführt wurde, wenn der intraabdominale Druck höher ist als der Beckendruck.

**16.** Das System nach Anspruch 15, wobei das Datenverarbeitungsmodul so konfiguriert ist, dass es einen gleichge- richteten Wert $q^*(i) = \dfrac{1}{1 + q(i)}$ verarbeitet, der Werte in [0,1] annimmt, und um Informationen über eine falsche PFM-Kontraktion zu erkennen und auszugeben, wenn $q^*(i) > 0,5$ ist.

**17.** Das System nach einem der Ansprüche 14 bis 16, das ferner einen Computer umfasst, der so konfiguriert ist, dass er mit dem Datenverarbeitungsmodul der Sondenvorrichtung kommuniziert, um die vom Datenverarbeitungsmodul erzeugten Ausgabedaten an den Computer der Person zu übermitteln, wobei die Sondenvorrichtung ferner einen Sender umfasst, der so konfiguriert ist, dass er die vom Datenverarbeitungsmodul erzeugten Ausgabedaten an den Computer sendet.

**18.** Das System nach einem der Ansprüche 1 bis 13, wobei das Datenverarbeitungsmodul (202) ferner so konfiguriert ist, dass es einen Satz von Differenzdruckdaten $\Delta Pmz(k)$ und $\Delta Pdt(k)$ von Messpunkten (k) während einer willentlichen Beckenbodenmuskelkontraktion registriert,

wobei das Datenverarbeitungsmodul so konfiguriert ist, dass es eine Ergebnis-Verhältnisfunktion RRF berechnet, die definiert ist als RRF (k) = Func (ΔPmz(k) / ΔPdt(k)), wobei RFF(k) eine Zahlenreihe ist und jede Zahl für eine Qualität der Beckenbodenmuskelkontraktion steht.

19. Das System nach einem der Ansprüche 1 bis 13, wobei das Datenverarbeitungsmodul (202) ferner so konfiguriert ist, dass es während einer Kalibrierungsphase einen Satz von Differenzdruckdaten ΔPmz(kv) und ΔPdt(kv) von Messpunkten (kv) während willentlicher intraabdominaler Kontraktionsmanöver registriert und einen Satz von Differenzdruckdaten ΔPmz(kh) und ΔPdt(kh) von Messpunkten (kh) während willentlicher Beckenbodenmuskelkontraktionen registriert,

- das Datenverarbeitungsmodul ist auch so konfiguriert, dass es zwei mehrdimensionale Reihen erzeugt, wobei eine erste Reihe auf bekannten Daten zu intraabdominalen Kontraktionsmanövern basiert: {(ΔPmz(i) , ΔPdt(i), δi)}, wobei i Indizes von Messpunkten sind, die während intra-abdominaler Kontraktionsmanöver registriert wurden, und δi= 1; und eine zweite Reihe, die auf Beckenbodenmuskelkontraktionsdaten basiert: {(ΔPmz(j), ΔPdt(j), δj)}, wobei j Indizes von Messpunkten sind, die während Beckenbodenmuskelkontraktionen registriert wurden, und δj = 0, und um den registrierten Satz von Differenzdruckdaten in eine erste charakterisierende Funktion CF1= Σ$_k$(ΔPmz(k) / ΔPdt(k) - c1x) zu verarbeiten,
wobei ein optimaler Wert c1xop von c1x so gewählt wird, dass CH1 für δi=0 positiv ist, und dass CF1 für δi=1 negativ ist, wobei der Schwellenwert (c1) durch den genannten optimalen Wert c1xop von clx geladen wird,
- wobei das Datenverarbeitungsmodul außerdem so konfiguriert ist, dass es den Schwellenwert (c1) wie er aus der internen Kalibrierungsphase erhalten wurde, speichert.

20. Das System nach Anspruch 19, wobei das Datenverarbeitungsmodul ferner so konfiguriert ist, dass es den registrierten Satz von Differenzdruckdaten zu einer zweiten charakterisierenden Funktion CF2 verarbeitet, die wie folgt definiert ist:

$$CF2 = \frac{1}{1 + e^{\left(c2\frac{\Delta Pmz(i)}{\Delta Pdt(i)} - c1\right)}}$$

Und ein Aufwandsoptimierungsalgorithmus wird anhand der folgenden Aufwandsfunktion definiert:

$$Cost(c_{1x}, c_{2x}) = ||CF2 - \delta i||$$

wobei die Funktion ‖*‖ eine metrische Abstandsfunktion ist und

- wobei das Datenverarbeitungsmodul ferner so konfiguriert ist, dass es die optimalen Parameter c1 und c2 speichert, die durch den Optimierungsalgorithmus erhalten wurden,
- eine Schnittstelle, die mit dem Datenverarbeitungsmodul kommuniziert, wobei die Schnittstelle so konfiguriert ist, dass sie die Daten vom Datenverarbeitungsmodul empfängt und das Auftreten einer falschen Kontraktion

meldet, wenn $\mathrm{CF2(j)} = \dfrac{1}{1 + \mathrm{EXP}\left[c2(\Delta Pmz(j) / \Delta Pdt(j) - c1)\right]}$ über 0,5 liegt.

## Revendications

1. Système de surveillance des contractions des muscles du plancher pelvien (MPP) effectuées par un sujet, notamment une femme, le système comprenant :

- un dispositif de sonde du plancher pelvien (100) configuré pour être positionné à l'intérieur du canal vaginal et en contact avec la surface vaginale interne du sujet, ladite sonde comprenant un corps avec une extrémité distale et une extrémité proximale, une surface externe, et au moins deux sections, une première section (106) étant à l'extrémité proximale de la sonde pour entrer en contact avec la zone médiane du canal vaginal et une seconde section (108) étant positionnée à l'extrémité distale de la sonde pour entrer en contact avec la zone distale du canal vaginal, chacune desdites première et deuxième sections ayant une forme de bulbe, la première section comprenant au moins un premier capteur adapté pour mesurer la pression appliquée par la surface vaginale interne à la surface externe de la sonde au niveau de la zone médiane du canal vaginal, et la deuxième section comprenant au moins un second capteur adapté pour mesurer la pression appliquée par la surface vaginale interne à la surface externe de la sonde au niveau d'une zone distale,

- un module de traitement des données (202) configuré pour déterminer une première pression vaginale différentielle dans la zone médiane de la cavité du plancher pelvien, notée ΔPmz, et une seconde pression différentielle dans la zone distale, notée ΔPdt, la première et la seconde pressions différentielles ayant pour référence des valeurs de pression mesurées dans un état de relaxation de l'utilisateur,

et le module de traitement des données (202) est configuré pour calculer au moins un rapport ΔPmz/ΔPdt et le comparer à une valeur seuil (c1),

dans lequel le module de traitement des données est en communication avec un dispositif utilisateur pour notifier, dans le cas où le rapport est inférieur à ladite première valeur seuil, une contraction sous-optimale des muscles du plancher pelvien.

2. Système selon la revendication 1, dans lequel le dispositif de sonde du plancher pelvien (100) s'étend le long d'un axe principal (X), dans lequel le premier capteur présente une direction principale de détection le long d'une première direction transversale (W1) et le second capteur présente une direction principale de détection le long d'une seconde direction transversale (Y2), dans laquelle ladite seconde direction transversale est sensiblement perpendiculaire à la première direction transversale, et de préférence, la première et la seconde directions transversales (W1, Y2) sont sensiblement perpendiculaires à l'axe principal (X).

3. Système selon l'une quelconque des revendications 1 à 2, dans lequel ladite première section (106) est séparée de ladite deuxième section (108) par une section de taille (107), ladite section de taille ayant une section transversale (D7) de taille inférieure à 75 % de la taille de la section transversale (D6) de ladite première section (106) et dans laquelle la section de taille présente une flexibilité permettant un désalignement de ladite deuxième section (108) par rapport à ladite première section (106).

4. Système selon l'une quelconque des revendications 1 à 3, dans lequel le dispositif de sonde du plancher pelvien (100) comprend une troisième section (104) disposée à l'extrémité proximale du dispositif, et la troisième section (104) est séparée de la première section (106) par une partie de taille de base (105).

5. Système selon la revendication 2, dans lequel la troisième section (104) présente une section transversale oblongue avec une plus grande dimension le long d'une orientation de base notée WO, qui est parallèle à la première direction transversale (W1), de sorte que l'utilisateur est incité à placer le dispositif avec l'orientation de base alignée avec sa direction antéropostérieure.

6. Système selon l'une quelconque des revendications 1 à 5, dans lequel le dispositif de sonde du plancher pelvien (100) comprend un premier élément de batterie logé dans la première section (106) et un second élément de batterie logé dans la deuxième section (108).

7. Système selon l'une quelconque des revendications 3 à 6, dans lequel la première section (106) et la deuxième section (108) sont attachées ensemble par un anneau de liaison (6) disposé au niveau de la section de la taille (107), l'anneau de liaison (6) étant entouré d'un collier (7).

8. Système selon l'une quelconque des revendications 2 à 5, dans lequel la première section (106) comprend deux premières coquilles complémentaires (11,12) qui, une fois assemblées, forment le corps bulbeux, avec un premier joint d'assemblage disposé généralement sur un plan perpendiculaire à la première direction transversale W1.

9. Système selon l'une quelconque des revendications 2 à 5, dans lequel la deuxième section (108) comprend deux secondes coquilles complémentaires (21, 22) qui, une fois assemblées, forment le corps bulbeux, avec un second joint d'assemblage disposé généralement sur un plan perpendiculaire à la seconde direction transversale Y2.

10. Système selon l'une quelconque des revendications 1 à 9, dans lequel la première section (106) présente une section transversale sensiblement circulaire (D6), avec un diamètre extérieur ne dépassant pas 30 mm, de préférence ne dépassant pas 28 mm.

11. Système selon l'une quelconque des revendications 1 à 10, dans lequel les premier et second capteurs sont des capteurs électroniques choisis parmi les capteurs électromécaniques, les capteurs de pression ou les capteurs de force.

**12.** Système selon l'une quelconque des revendications 1 à 11, dans lequel la première section est centrée sur une première position axiale située à une première distance de l'extrémité distale dudit corps de sonde, la première distance étant comprise entre 6 et 8 cm, de préférence 7cm, et la deuxième section est centrée sur une seconde position axiale située à une seconde distance de l'extrémité distale dudit corps de sonde, la seconde distance étant comprise entre 2,5 et 3,5 cm, de préférence 3cm.

**13.** Système selon l'une quelconque des revendications 1 à 12, dans lequel le dispositif comprend une enveloppe étanche au fluide (8), ladite enveloppe étant constituée d'un élastomère biocompatible, de préférence un silicone de qualité médicale.

**14.** Système selon l'une quelconque des revendications 1 à 13, dans lequel le module de traitement des données (202) est configuré pour enregistrer un ensemble de données de pressions différentielles $\Delta Pmz(k_v)$ et $APdt(k_v)$ de points d'échantillonnage pendant des contractions de Valsalva (volontaires) connues $(K_v)$, et pour enregistrer un ensemble de données de pressions différentielles $\Delta Pmz(k_h)$ et $\Delta Pdt(k_h)$ de points d'échantillonnage pendant des contractions saines (volontaires) connues $(k_h)$, pendant la phase d'étalonnage interne, ledit module de traitement des données étant également configuré pour créer deux tableaux multidimensionnels, un premier tableau basé sur des données de contractions de Valsalva connues : $\{(\Delta Pmz(i), \Delta Pdt(i), \delta i)\}$, où i sont des indices de points d'échantillonnage enregistrés pendant une contraction de Valsalva (volontaire) connue et $\delta i = 1$ ; et un second tableau basé sur des données de contraction saine (volontaire) connue : $\{(\Delta Pmz(j), \Delta Pdt(j), \delta j)\}$, où j sont des indices de points d'échantillonnage enregistrés pendant une contraction saine et $\delta j = 0$, et pour traiter l'ensemble des données de pressions différentielles enregistrées dans un algorithme d'optimisation des coûts standard conformément à la fonction de coût suivante :

$$Cost\,(c1) = \left\| \boldsymbol{\sigma}\left(c1, \Delta Pmz\,(k), \Delta Pdt\,(k)\right) - \delta_k \right\|$$

où k représente tous les points d'échantillonnage soit pendant les contractions de Valsalva $(k_v)$ soit pendant les contractions saines $((k_h)$,
dans lequel la fonction $\|*\|$ *est une fonction de distance métrique,* et
où $\sigma(i)$ est calculé comme suit :

$$\sigma(i) = \frac{1}{1 + e^{\left(\frac{\Delta Pmz(i)}{\Delta Pdt(i)} - 1\right)}}$$

- ledit module de traitement des données étant, en outre, configuré pour stocker le paramètre optimal c1 obtenu à partir de l'algorithme d'optimisation et pour mesurer les pressions différentielles $\Delta Pmz$ et $\Delta Pdt$ des MPP d'un sujet pendant la phase d'entraînement avec lesdits réseaux de capteurs desdites au moins deux sections de la sonde et, à l'aide du module de traitement des données, pour calculer le rapport $\Delta Pmz / \Delta Pdt$,
- une interface en communication avec le module de traitement des données, ladite interface étant configurée pour recevoir des données du module de traitement des données indiquant que la contraction est une contraction incorrecte des MPP ou une contraction de Valsalva lorsque le rapport est inférieur à la valeur seuil cl, et configurée, en outre, pour notifier au sujet l'apparition d'une contraction de Valsalva.

**15.** Système selon la revendication 14, dans lequel ledit module de traitement des données est, en outre, configuré pour traiter une valeur q de la qualité de la contraction du muscle du plancher pelvien à chaque point d'échantillonnage (i), $q(i)$ étant calculé comme suit :

$$q(i) = e^{c2 * \left(\frac{\Delta Pmz(i)}{\Delta Pdt(i)} - c1\right)}$$

où cl est affecté d'une valeur comprise entre 0,8 et 4, ou entre 1 et 3, ou entre 1 et 2, ou est égal à 1,2 ;
où c2 se voit attribuer une valeur comprise entre -20 et +20, ou entre -10 et +10, ou entre 0 et 5 ou égale à 2 ; et
dans lequel ledit module de traitement des données est configuré pour détecter et émettre des informations indiquant qu'une contraction incorrecte des MPP a été effectuée lorsque la pression intra-abdominale est supérieure à la pression pelvienne.

**16.** Système selon la revendication 15, dans lequel ledit module de traitement des données est configuré pour traiter une

valeur rectifiée $q^*(i) = \dfrac{1}{1+q(i)}$ qui prend des valeurs dans [0,1], et pour détecter et émettre des informations sur une contraction incorrecte des MPP lorsque $q^*(i) > 0,5$.

17. Système selon l'une quelconque des revendications 14 à 16, comprenant, en outre, un dispositif informatique configuré pour communiquer avec le module de traitement des données du dispositif de sonde afin de transmettre les données de sortie générées par le module de traitement des données au dispositif informatique du sujet, le dispositif de sonde comprenant, en outre, un émetteur configuré pour envoyer les données de sortie générées par le module de traitement des données au dispositif informatique.

18. Système selon l'une quelconque des revendications 1 à 13, dans lequel le module de traitement des données (202) est, en outre, configuré pour enregistrer un ensemble de données de pressions différentielles $\Delta Pmz(k)$ et $\Delta Pdt(k)$ de points d'échantillonnage (k) pendant une contraction volontaire des muscles du plancher pelvien, dans lequel ledit module de traitement des données est configuré pour calculer une fonction de rapport de résultat RRF définie comme RRF(k)= Func ($\Delta Pmz(k)$ / $\Delta Pdt(k)$), où RFF(k) est une série de nombres, chaque nombre étant représentatif d'une qualité de la contraction des muscles du plancher pelvien.

19. Système selon l'une quelconque des revendications 1 à 13, dans lequel le module de traitement des données (202) est, en outre, configuré pour enregistrer, pendant une phase d'étalonnage, un ensemble de données de pressions différentielles $\Delta Pmz(kv)$ et $\Delta Pdt(kv)$ de points d'échantillonnage (kv) pendant des manœuvres de contraction intra-abdominale volontaire, et pour enregistrer un ensemble de données de pressions différentielles $\Delta Pmz(kh)$ et $4\Delta Pdt(kh)$ de points d'échantillonnage (kh) pendant des contractions musculaires volontaires du plancher pelvien,

 - ledit module de traitement des données est également configuré pour créer deux tableaux multidimensionnels, un premier tableau basé sur les données connues relatives aux manœuvres de contraction intra-abdominale: $\{(\Delta Pmz(i) , \Delta Pdt(i), \delta i)\}$, où i sont des indices de points d'échantillonnage enregistrés pendant les manœuvres de contraction intra-abdominale et $\delta i = 1$ ; et un second tableau basé sur les données de contraction des muscles du plancher pelvien : $\{(\Delta Pmz(j), \Delta Pdt(j), \delta j)\}$, où j sont des indices de points d'échantillonnage enregistrés pendant les contractions des muscles du plancher pelvien et $\delta j = 0$, et de traiter l'ensemble enregistré des données de pressions différentielles dans une première fonction de caractérisation CF1= $\Sigma_k(\Delta Pmz(k) / \Delta Pdt(k) - c1x)$, où une valeur optimale c1op de c1x est choisie de sorte que CF1 soit positive pour $\delta i=0$, et que CF1 soit négative pour $\delta i=1$, la **valeur seuil** (c1) étant chargée par ladite valeur optimale **c1xop** de clx,
 - ledit module de traitement des données est, en outre, configuré pour stocker la **valeur seuil** (c1) telle qu'obtenue lors de la phase d'étalonnage interne.

20. Système selon la revendication 19, dans lequel ledit module de traitement des données est, en outre, configuré pour traiter l'ensemble enregistré de données de pressions différentielles dans une seconde fonction de caractérisation CF2 définie comme suit :

$$CF2 = \dfrac{1}{1 + e^{\left(c2\frac{\Delta Pmz(i)}{\Delta Pdt(i)}-c1\right)}}$$

un algorithme d'optimisation des coûts est défini selon la fonction des coûts suivante :

$$Cost(c_{1x}, c_{2x}) = ||CF2 - \delta i||$$

dans lequel la fonction $\|*\|$ est une fonction de distance métrique et

 - ledit module de traitement des données est, en outre, configuré pour stocker les paramètres optimaux c1 et c2 tels qu'obtenus à partir de l'algorithme d'optimisation,
 - une interface en communication avec le module de traitement des données, ladite interface étant configurée pour recevoir des données du module de traitement des données et notifier l'apparition d'une contraction incorrecte lorsque $CF2(j) = \dfrac{1}{1 + EXP[c2(\Delta Pmz(j) / \Delta Pdt(j) - c1)]}$ est supérieur à 0, 5 .

**FIG. 1**

**FIG. 2**

Insert A Probe Of A Pelvic Floor Probe Device
Within A Pelvic Cavity Of A User — 302

Provide A Set Of Targets To Perform PFM Exercise — 304

Collect The Pressure Data Related To Pelvic
Floor Muscle Contraction Of The User From A First
And Second Sensor Of The Device — 306

Calculate, At A Data Processing Module, A Phase
Contraction Of Muscles
$s(i)$ Using $\exp([P_a(i)/P_b(i) - c_1] * c_2$ — 308

Notify The User In Case Of Incorrect Pelvic Floor
Muscle Contraction Via A User Interface — 310

**FIG. 3**

**FIG. 4**

ΔPmz > ΔPdt    ΔPmz < ΔPdt

ΔPmz

ΔPdt

ΔPdt

ΔPmz

## FIG. 5

X'    X

β

67    6

84

66    87

67

## FIG. 6

FIG. 7

FIG. 8

FIG. 9

FIG. 10

## REFERENCES CITED IN THE DESCRIPTION

**Patent documents cited in the description**

- US 2015196802 A
- US 20180264317 A1

- CN 110538432 A